# EUROPEAN PATENT SPECIFICATION

(11) **EP 4 074 814 B1**
(45) Date of publication and mention of the grant of the patent: **15.01.2025**
(21) Application number: 21205920.8
(22) Date of filing: 02.11.2021
(51) Int. Cl.: C12M 1/12, C12M 1/32, C12M 1/00

(54) **CELL AND MEDICAMENT DISPENSING DEVICE FOR DRUG SCREENING AND METHOD THEREOF**
ZELLE UND MEDIKAMENTENABGABEVORRICHTUNG FÜR DAS ARZNEIMITTEL-SCREENING UND VERFAHREN DAZU
DISPOSITIF DE DISTRIBUTION DE CELLULES ET DE MÉDICAMENTS POUR CRIBLAGE DE MÉDICAMENTS ET PROCÉDÉ ASSOCIÉ

(43) Date of publication of application: 19.10.2022
(73) Proprietor: Drsignal Biotechnology Co., Ltd, New Taipei City (TW)
(72) Inventor: MI, Hsin Wu, New Taipei City (TW); HUANG, Hsin Fei, New Taipei City (TW)
(74) Representative: Cabinet Chaillot

(56) References cited:
- US-A1- 2002 012 611
- US-A1- 2002 176 803
- US-A1- 2011 220 239
- US-A1- 2019 195 901

## Description

### 1. Field of the Invention

The present invention relates to an automation equipment for biology and chemistry laboratories, especially to a device and a method configured to dispense minute amount of cells or minute amount of medicaments into different wells of a culture plate.

### 2. Description of the Prior Arts

To perform drug screening, first dispense minute amount of cells into wells of a culture plate where the cells are then cultivated for several days. When the cultivation is completed, manually prepare different kinds of medicament liquids in different containers, manually draw up and dispense precise amount of each medicament liquid into the wells with the cultivated cells one after another with a pipette.

However, a typical culture plate has dozens of wells, and there are several medicament liquids needed to be dispensed. As a result, simple and repetitive dispensing operation needs to be repeated for hundreds of times for each culture plate, making drug screening a long and laborious task.

Additionally, a disposable tip is mounted on a front end of the pipette, and the tip needs to be replaced before dispensing a different kind of cell or medicament liquid to prevent contamination. Replacement of the tip further increases the time and labor for drug screening.

In addition, an automated pipetting system is disclosed in US patent publication No. 2002/176803A1. A dispensing robot, method of controlling dispensing robot, and dispensing method are disclosed in US patent publication No. 2019/195901A1. A method for rapidly identifying useful chemicals in liquid sample is disclosed in US patent publication No. 2002/012611A1. A high density plate filler is disclosed in US patent publication No. 2011/220239A1.

The main objective of the present invention is to provide a cell and medicament dispensing device for drug screening and method thereof to automate the complex process of drug screening.

The cell and medicament dispensing device for drug screening of claim 1 is configured to inject multiple solutions into multiple solution recesses of a transfer plate. Then, liquid inside each of the solution recesses is dispensed into multiple wells of a cell culture plate with a pipette. At least one pipette-tip is detachably connected to a bottom of the pipette. The cell and medicament dispensing device comprises a base, a transfer plate serving mechanism, a transfer plate positioning mechanism, an injection mechanism, a cell culture plate positioning mechanism, and a dispensing mechanism. The base has a transfer plate entrance area, a receiving area, and a dispensing area thereon. The transfer plate serving mechanism is mounted on the base and is adjacent to the transfer plate entrance area. The transfer plate serving mechanism is configured to accommodate the transfer plate and is capable of moving the transfer plate to the transfer plate entrance area of the base. The transfer plate serving mechanism has a plate stacking bracket, a plate dropping opening, two plate-locking clamps, a plate feeder, and a plate lifting actuator. The plate stacking bracket forms a stacking space. The stacking space is configured to accommodate multiple spare transfer plates that are stacked vertically. The plate dropping opening is formed in a bottom of the plate stacking bracket. The spare transfer plates are capable of dropping through the plate dropping opening. The two plate-locking clamps are each mounted on a respective side of two opposite sides of the plate dropping opening. The two plate-locking clamps are capable of moving toward each other to prevent the spare transfer plates from passing through the plate dropping opening. The plate feeder is mounted under the plate dropping opening and is configured to receive the transfer plates dropped through the plate dropping opening. The plate feeder is movable to the transfer plate entrance area of the base, and has a plate lifting opening formed through two opposite sides of the plate feeder. The plate lifting actuator is mounted under the plate lifting opening and is capable of moving upward to protrude through the plate lifting opening. The transfer plate positioning mechanism is mounted on the base and is adjacent to the transfer plate serving mechanism. The transfer plate positioning mechanism has a positioning slider configured to connect with the transfer plate. The positioning slider is movably mounted on the base and selectively corresponds in position to the transfer plate entrance area, the receiving area, or the dispensing area. The plate feeder is configured to move to a position above the positioning slider to transfer the transfer plate to the positioning slider. The injection mechanism is mounted on the base and is adjacent to the transfer plate positioning mechanism. The injection mechanism has multiple injection heads. Each of the injection heads is in fluid communication with a respective one of the solutions and is capable of moving to the receiving area of the base. The positioning slider is configured to align any one of the solution recesses of the transfer plate to the injection head located in the receiving area such that said injection head injects one of the solutions into the corresponding solution recess. The cell culture plate positioning mechanism is mounted on the base and configured to connect with the cell culture plate. The cell culture plate positioning mechanism has a positioning seat and a primary positioning module. The positioning seat is mounted on the base and configured to accommodate the cell culture plate. The primary positioning module is mounted on the positioning seat and configured to clamp the cell culture plate. The dispensing mechanism is mounted on the base and has a dispensing seat. The dispensing seat is configured to fix the pipette and control aspirate operation and discharge operation of the pipette. The dispensing seat is movable relative to the base and capable of moving back and forth between a position above the dispensing area of the base and a position above the cell culture plate positioning mechanism. When the dispensing seat is above the dispensing area, the dispensing seat is configured to insert the at least one pipette-tip of the pipette into any one of the solution recesses of the transfer plate and executes aspirate operation of the pipette. The dispensing seat is configured to move to a position above the cell culture plate positioning mechanism and execute the discharge operation of the pipette to release the solution aspired in the pipette into one of the wells of the cell culture plate.

The cell and medicament dispensing method for drug screening of claim 8 comprises steps as follows:
(a) Transfer of cells or solutions: A transfer plate serving mechanism has a plate stacking bracket, a plate dropping opening, two plate-locking clamps, a plate feeder, and a plate lifting actuator. The plate stacking bracket forms a stacking space. The stacking space is configured to accommodate multiple spare transfer plates that are stacked vertically; the plate dropping opening is formed in a bottom of the plate stacking bracket. The spare transfer plates are capable of dropping through the plate dropping opening. The two plate-locking clamps are each mounted on a respective side of two opposite sides of the plate dropping opening. The two plate-locking clamps are capable of moving toward each other to prevent the spare transfer plates from passing through the plate dropping opening. The plate feeder is mounted under the plate dropping opening and is configured to receive a transfer plate dropped through the plate dropping opening. The plate feeder is movable to the transfer plate entrance area of the base and has a plate lifting opening formed through two opposite sides of the plate feeder. The plate lifting actuator is mounted under the plate lifting opening and is capable of moving upward to protrude through the plate lifting opening. The plate feeder of the transfer plate serving mechanism moves the transfer plate to a transfer plate entrance area on a base. The transfer plate has multiple solution recesses formed thereon. A positioning slider of a transfer plate positioning mechanism moves to the transfer plate entrance area and connects with the transfer plate located in the transfer plate entrance area. The plate feeder of the transfer plate serving mechanism moves to the position above the positioning slider to transfer the transfer plate to the positioning slider. The positioning slider moves the transfer plate to a receiving area on the base. An injection mechanism injects multiple solutions into the solution recesses of the transfer plate.
(b) Dispensing of cells or solutions: The positioning slider moves the transfer plate to a dispensing area on the base. A cell culture plate with multiple wells is ready at a position by the cell culture plate positioning mechanism. A dispensing mechanism moves a pipette back and forth between a position above the dispensing area on the base and a position above the cell culture plate on the positioning slider to dispense liquid in each of the solution recesses into the wells of the cell culture plate.

The advantage of the present invention is that liquids and cells for drug screening are automatically transferred to the solution recesses of the transfer plate by coordination among the transfer plate serving mechanism, the transfer plate positioning mechanism, and the injection mechanism. Afterwards, the liquid in each of the solution recesses is automatically dispensed into the wells of the cell culture plate by coordination between the transfer plate positioning mechanism and the dispensing mechanism. As a result, the process of drug screening could be automated to reduce labor and improve quality.

### IN THE DRAWINGS

Fig. 1 is a perspective view of a cell and medicament dispensing device for drug screening in accordance with the present invention;
Fig. 2 is a schematic top view of the cell and medicament dispensing device in Fig. 1;
Fig. 3 is a partial perspective view of the cell and medicament dispensing device in Fig. 1;
Fig. 4 is an exploded view of the cell and medicament dispensing device in Fig. 3;
Fig. 5 is a partial perspective view of the cell and medicament dispensing device in Fig. 1, showing a transfer plate serving mechanism, a transfer plate retriever, and a transfer plate positioning mechanism of the dispensing device;
Fig. 6 is a partial perspective view of the cell and medicament dispensing device in Fig. 1, showing the transfer plate serving mechanism, the transfer plate retriever, and the transfer plate positioning mechanism from another angle;
Fig. 7 is an exploded perspective view of the transfer plate serving mechanism, the transfer plate retriever, and the transfer plate positioning mechanism of the cell and medicament dispensing device in Fig. 6;
Fig. 8 and Fig. 9 are operational schematic side views of the cell and medicament dispensing device in Fig. 1, showing operating statuses of the transfer plate serving mechanism viewed along a transverse direction;
Figs. 10 to 13 are operational schematic cross sectional views of the cell and medicament dispensing device in Fig. 1, showing operating statuses of the transfer plate serving mechanism and the transfer plate positioning mechanism viewed along a longitudinal direction;
Figs. 14 to 16 are operational schematic side views of the cell and medicament dispensing device in Fig. 1, showing operating statuses of the transfer plate positioning mechanism and an injection mechanism;
Fig. 17 is a perspective view of a cell culture plate positioning mechanism of the cell and medicament dispensing device in Fig. 1;
Fig. 18 is an exploded perspective view of the cell culture plate positioning mechanism of the cell and medicament dispensing device in Fig. 17;
Figs. 19 and 20 are operational schematic side views of the cell culture plate positioning mechanism of the cell and medicament dispensing device in Fig. 17, showing operating statuses of the cell culture plate positioning mechanism viewed along the transverse direction;
Figs. 21 and 22 are operational schematic top views of the cell culture plate positioning mechanism of the cell and medicament dispensing device in Fig. 17, showing operating statuses of the cell culture plate positioning mechanism;
Figs. 23 and 24 are operational schematic cross sectional views of the cell culture plate positioning mechanism of the cell and medicament dispensing device in Fig. 17, showing operating statuses of a lid opener of the cell culture plate positioning mechanism;
Fig. 25 is a perspective view of a dispensing mechanism of the cell and medicament dispensing device in Fig. 1;
Fig. 26 is an exploded perspective view of the dispensing mechanism of the cell and medicament dispensing device in Fig. 25;

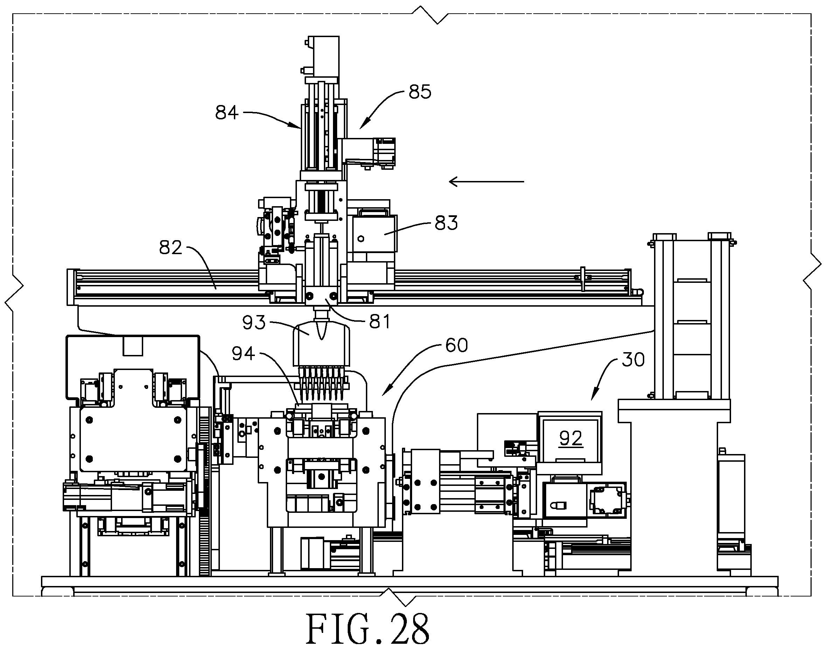

Figs. 27 to 29 are operational schematic side views of the cell and medicament dispensing device in Fig. 1, showing operating statuses of the dispensing mechanism viewed along the longitudinal direction;
Fig. 30 is an exploded perspective view of a pipette-tip feeder of the cell and medicament dispensing device in Fig. 1;
Figs. 31 and 32 are operational schematic side views of the cell and medicament dispensing device in Fig. 1, showing operating statuses of the dispensing mechanism and the pipette-tip feeder along the transverse direction;
Figs. 33 to 36 are operational schematic side views of the cell and medicament dispensing device in Fig. 1, showing operating statuses for replacing pipette-tips viewed along the transverse direction;
Fig. 37 is a schematic side view of the cell and medicament dispensing device in Fig. 1, showing a tip positioning module of the pipette-tip feeder being capable of moving upward and downward; and
Fig. 38 is a flow chart of a cell and medicament dispensing method for drug screening in accordance with the present invention.

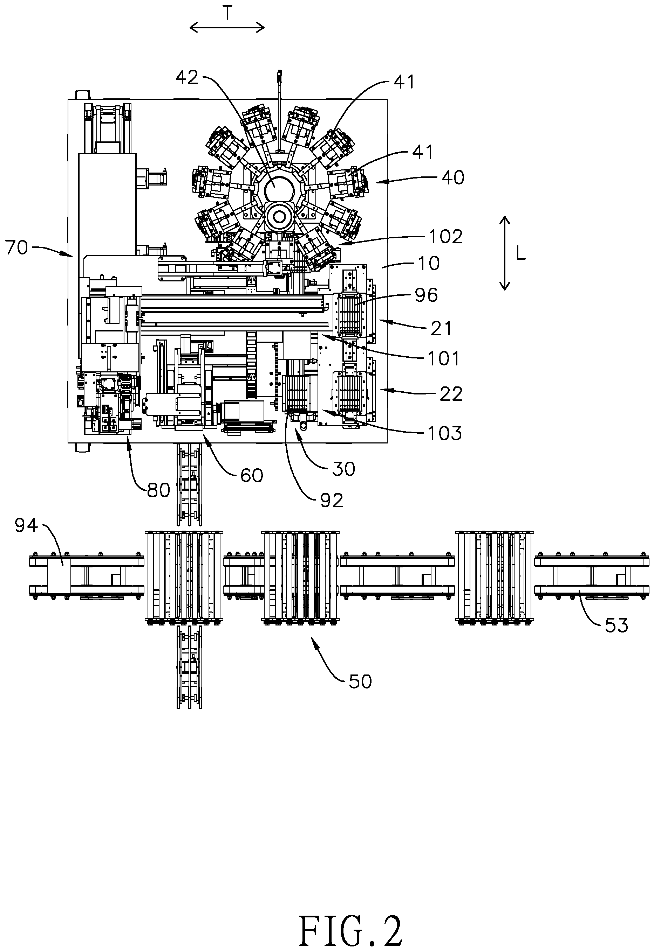

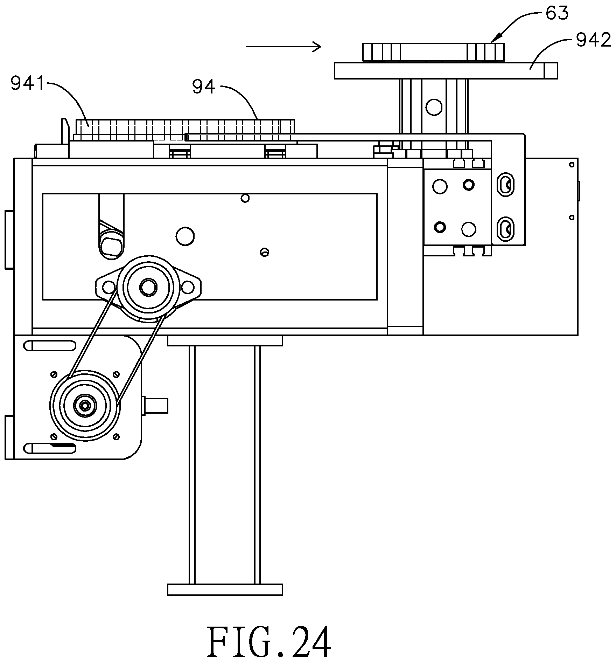

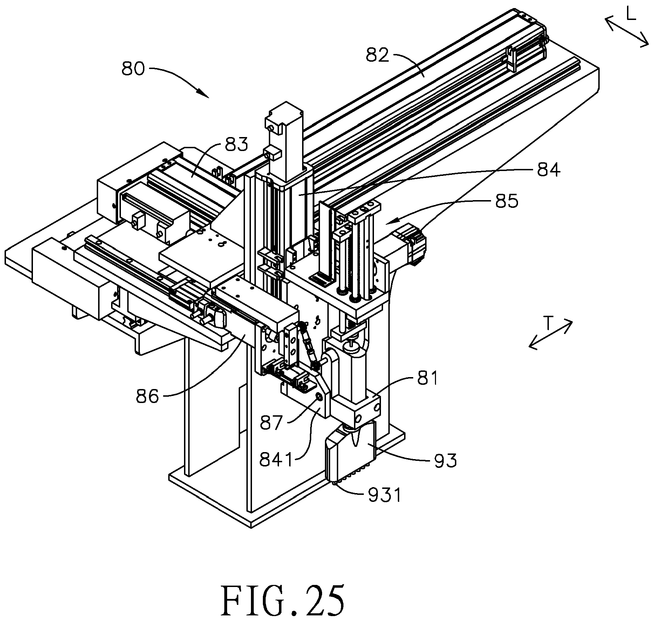

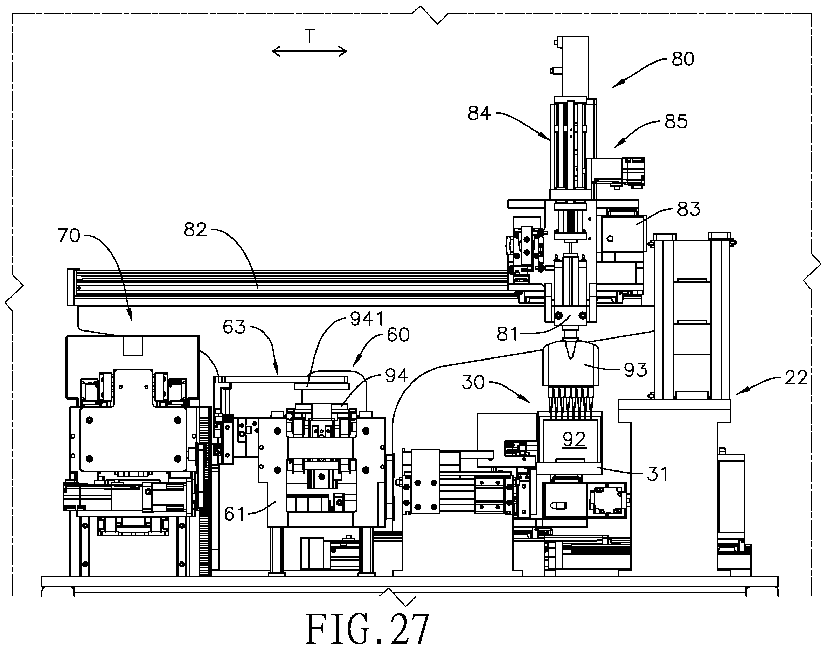

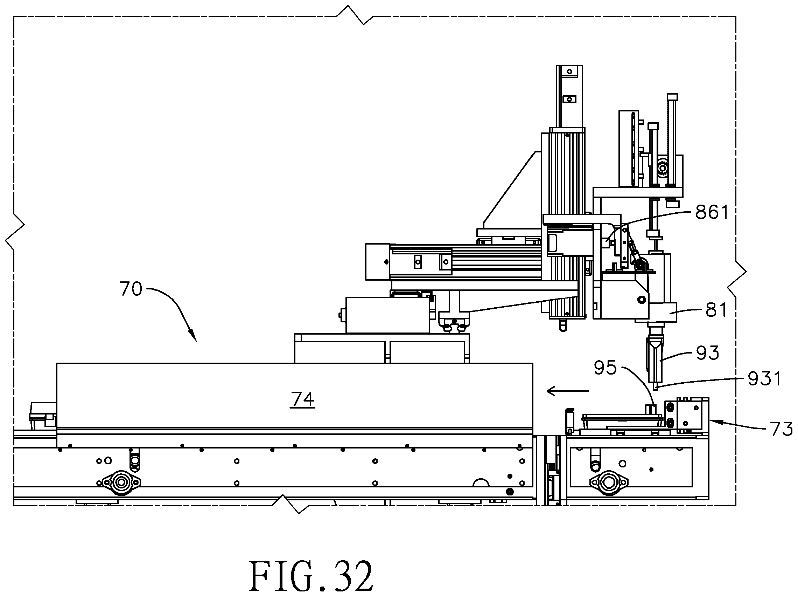

With reference to Figs. 2, 3, 6, 24, 25, and 27, a cell and medicament dispensing device for drug screening in accordance with the present invention is configured to inject multiple solutions 91 into multiple solution recesses 921 of a transfer plate 92, and then liquid inside each of the solution recesses 921 is dispensed into multiple wells 941 (as shown in Fig. 24) of a cell culture plate 94 with a pipette 93. Each of the solutions 91 comprises one single type of medicament or one single type of cells. At least one cylindrical pipette-tip 95 (as shown in Fig. 32) is detachably connected to a tip connector 931 (as shown in Fig. 25) at bottom of the pipette 93. The cell culture plate 94 preferably has an upper lid 942 detachably covering the wells 941.

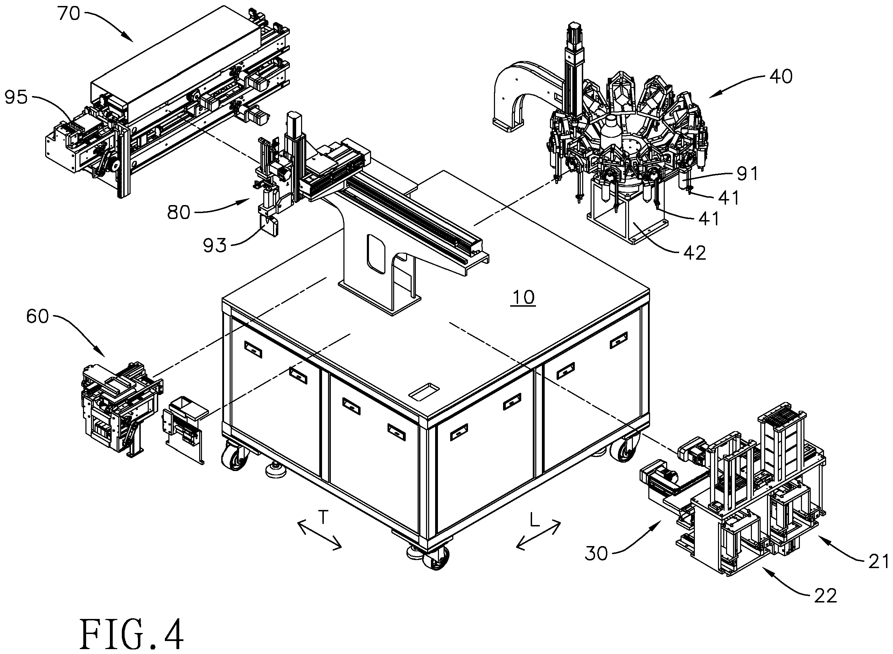

With reference to Figs. 2 to 4, the cell and medicament dispensing device has a base 10, a transfer plate serving mechanism 21, a transfer plate positioning mechanism 30, an injection mechanism 40, a cell culture plate positioning mechanism 60, and a dispensing mechanism 80. In the present embodiment, the cell and medicament dispensing device further has a cell culture plate conveyer 50, a pipette-tip feeder 70, and a transfer plate retriever 22.

The base 10 has a transfer plate entrance area 101, a receiving area 102, and a dispensing area 103 disposed thereon. The transfer plate entrance area 101, the receiving area 102, and the dispensing area 103 are disposed apart from each other along a longitudinal direction L of the base 10.

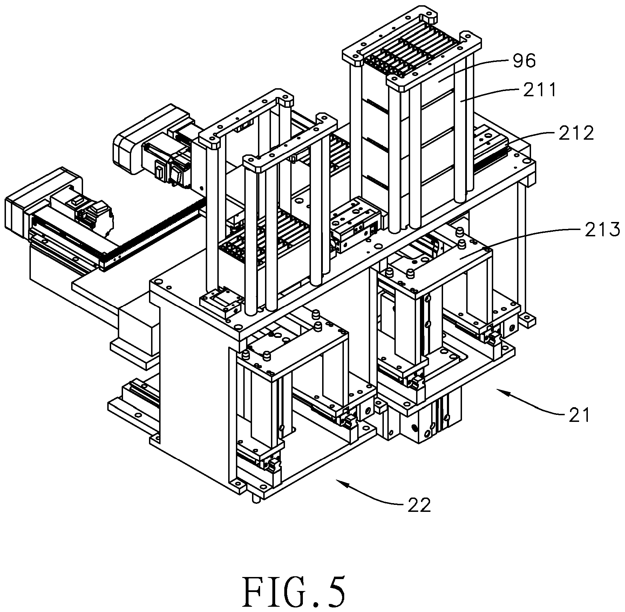

With reference to Figs. 5 to 7, the transfer plate serving mechanism 21 is mounted on the base 10 and is adjacent to the transfer plate entrance area 101. A transfer plate 92 is accommodated in the transfer plate serving mechanism 21, and the transfer plate serving mechanism 21 is capable of moving the transfer plate 92 to the transfer plate entrance area 101 of the base 10. In the preferred embodiment, the transfer plate serving mechanism 21 has a plate stacking bracket 211, two plate-locking clamps 212, a plate feeder 213, and a plate lifting actuator 214.

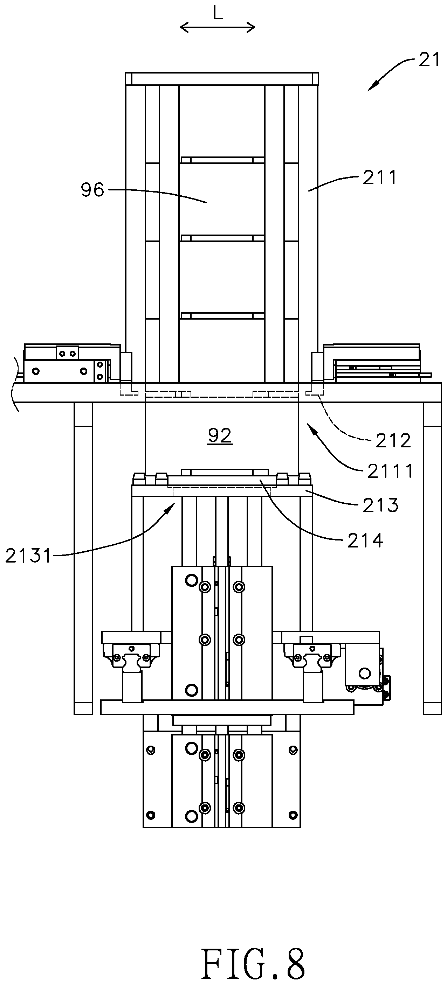

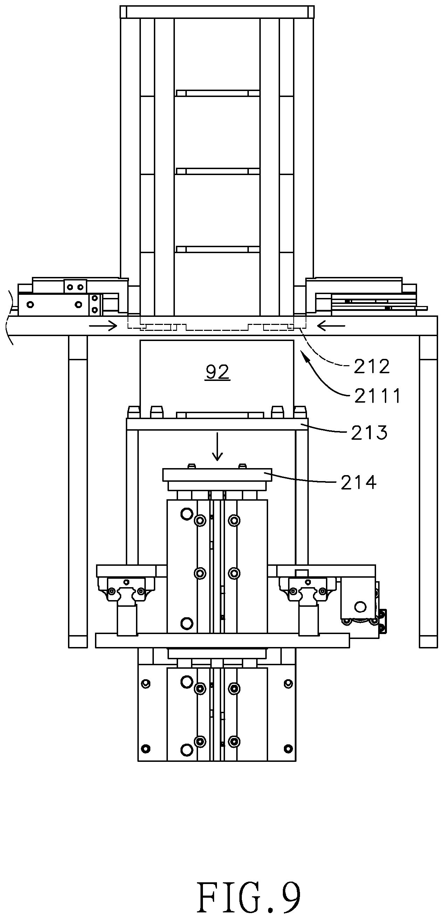

With reference to Figs. 8 and 9, the plate stacking bracket 211 forms a stacking space. The stacking space is configured to accommodate multiple spare transfer plates 96 that are stacked vertically. A plate dropping opening 2111 is formed in a bottom of the plate stacking bracket 211. The spare transfer plates 96 in the stacking space are capable of dropping down through the plate dropping opening 2111.

Each of the two plate-locking clamps 212 is mounted on a respective side of two opposite sides of the plate dropping opening 2111. The two plate-locking clamps 212 are capable of moving toward each other to prevent the spare transfer plates 96 from passing through the plate dropping opening 2111.

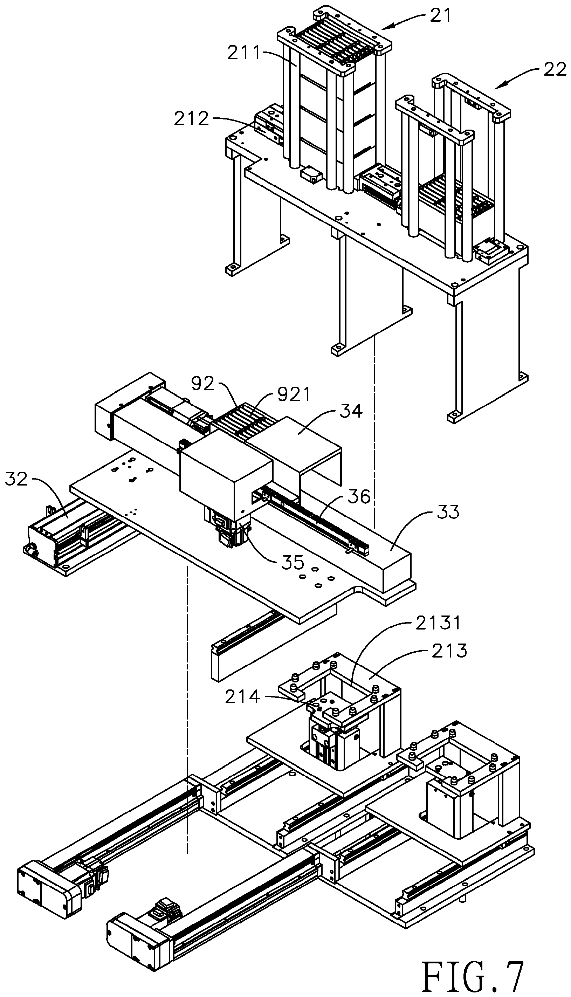

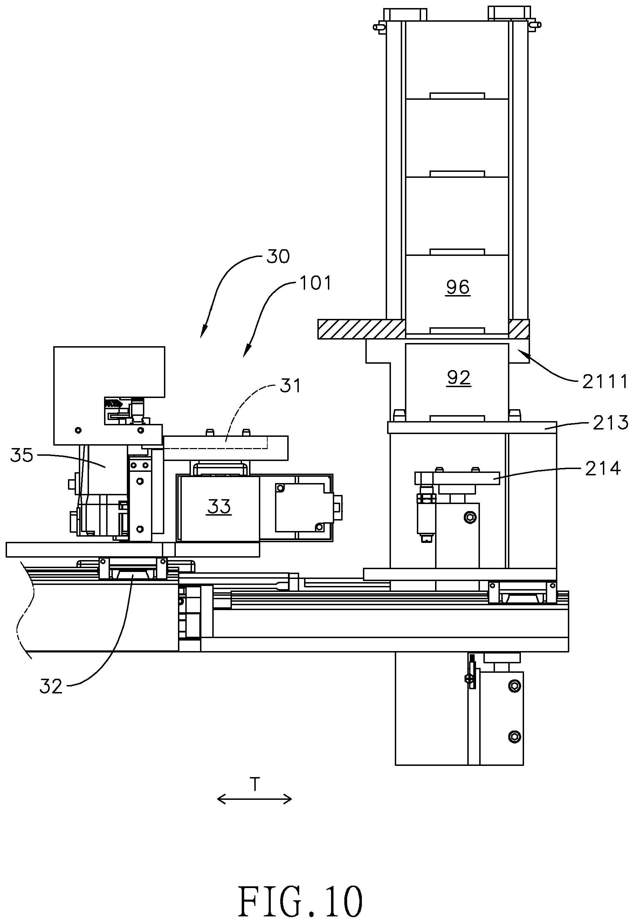

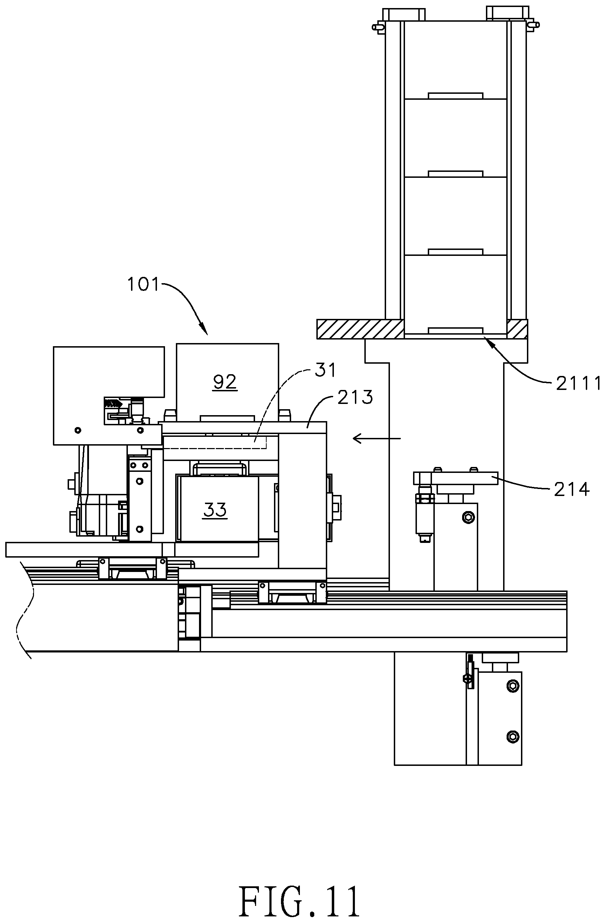

The plate feeder 213 is mounted under the plate dropping opening 2111 and is configured to receive the transfer plate 92 or the spare transfer plates 96 dropping down from the plate dropping opening 2111. With reference to Figs. 10 and 11, the plate feeder 213 is capable of moving to the transfer plate entrance area 101 on the base 10. With reference to Figs. 8 and 9, the plate feeder 213 has a plate lifting opening 2131 which is formed through two opposite sides of the plate feeder 213. With reference to Figs. 7 and 8, the plate lifting actuator 214 is mounted under the plate lifting opening 2131 and capable of moving upward to protrude through the plate lifting opening 2131.

The transfer plate positioning mechanism 30 is mounted on the base 10 and adjacent to the transfer plate serving mechanism 21. The transfer plate positioning mechanism 30 has a positioning slider 31, a first plate linear module 32, a second plate linear module 33, a sliding plate-cover 34, a cover driving motor 35, and a cover gear rack 36.

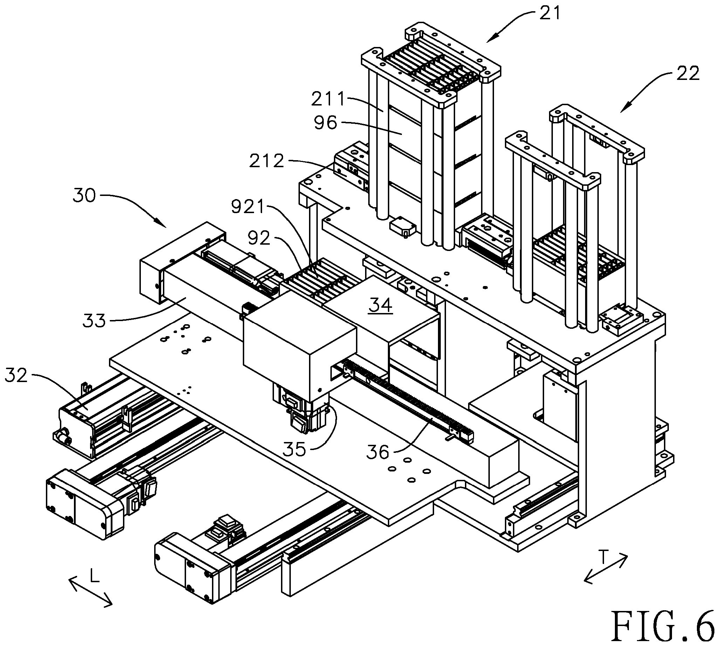

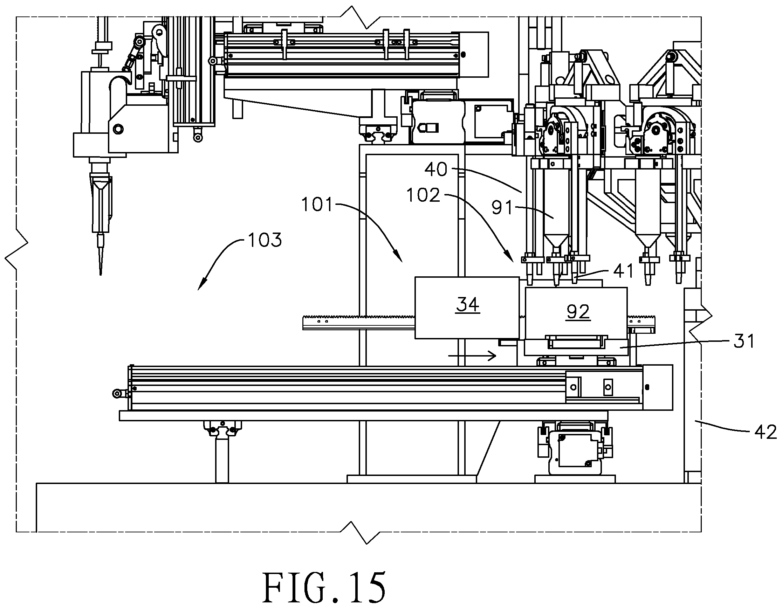

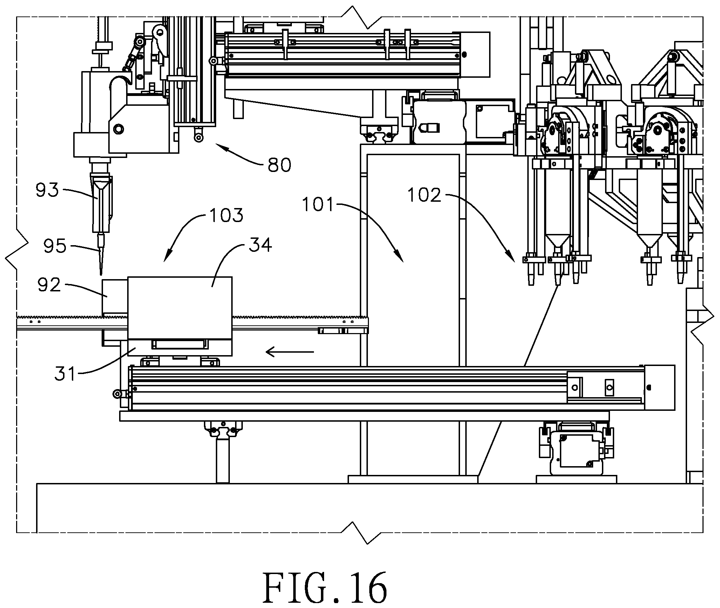

With reference to Figs. 6, 7, and 14 to 16, the positioning slider 31 is configured to connect with the transfer plate 92. The positioning slider 31 is movably mounted on the base 10 and selectively corresponds in position to the transfer plate entrance area 101, the receiving area 102, or the dispensing area 103.

To be specific, the first plate linear module 32 is mounted on the base 10 and extends along a transverse direction T of the base 10. The second plate linear module 33 is mounted on the first plate linear module 32 and extends along the longitudinal direction L of the base 10. The positioning slider 31 is mounted on the second plate linear module 33 such that the positioning slider 31 is movable in both the longitudinal direction L and transverse direction T.

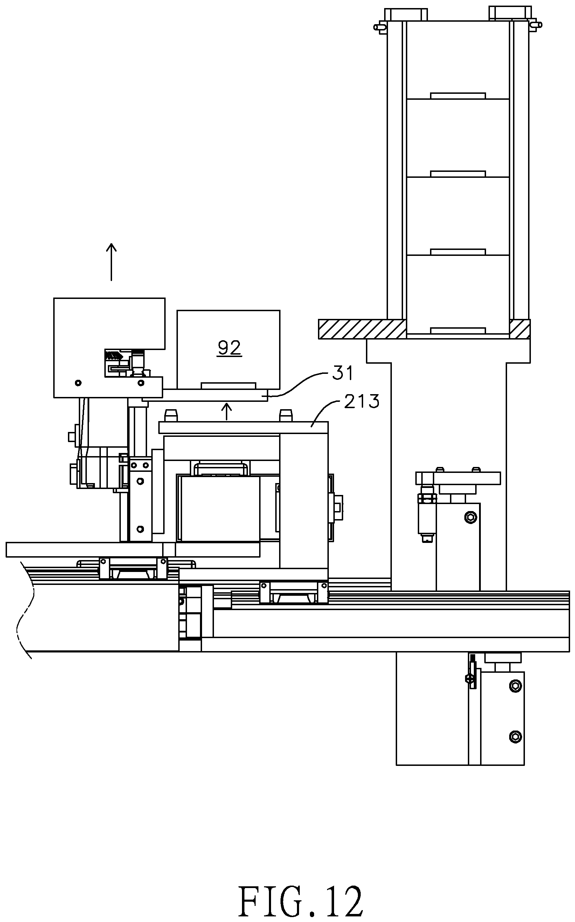

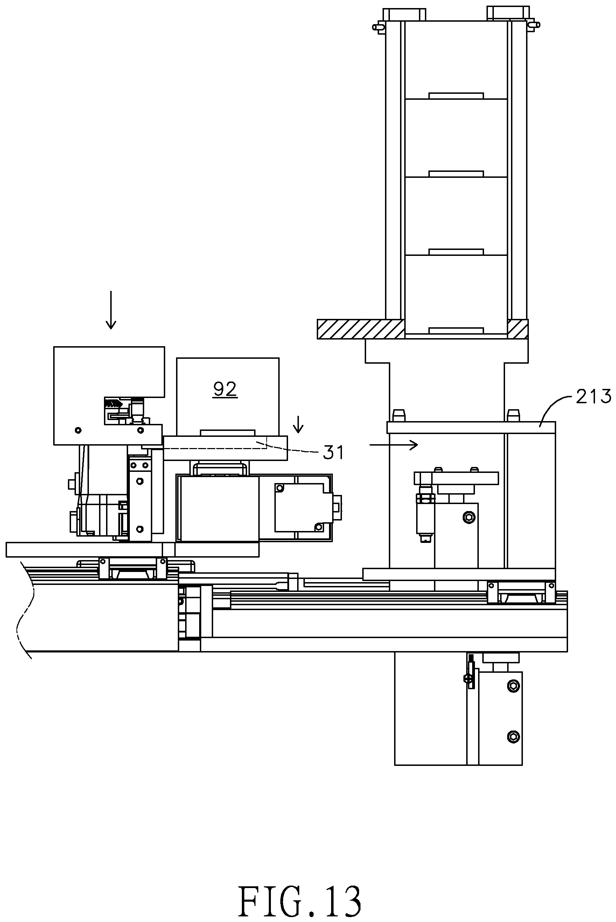

In the preferred embodiment, the plate feeder 213 of the transfer plate serving mechanism 21 is movable to a position above the positioning slider 31, and the positioning slider 31 is capable of moving upward and downward such that the transfer plate 92 can be transferred from the plate feeder 213 to the positioning slider 31. Detailed operating steps of the positioning slider 31 are as described below.
1. With reference to Figs. 10 to 11, the positioning slider 31 moves to the transfer plate entrance area 101, and the plate feeder 213 moves to the position above the positioning slider 31.
2. With reference to Figs. 11 and 12, the positioning slider 31 moves upward to protrude through the plate feeder 213 via the plate lifting opening 2131 such that the transfer plate 92 rested on the plate feeder 213 is lifted by the positioning slider 31 and connected with the positioning slider 31.
3. With reference to Figs. 12 and 13, the plate feeder 213 moves away from the position above the positioning slider 31, and then the positioning slider 31 moves downward to return to its original height position.

With reference to Figs. 6, 15, and 16, the cover driving motor 35 is mounted on the positioning slider 31. The cover gear rack 36 is mounted on the positioning slider 31, is movable along the longitudinal direction L, and is coupled to an output axis of the cover driving motor 35. The sliding plate-cover 34 is fixed to the cover gear rack 36 such that when the cover gear rack 36 moves relative to the positioning slider 31, the sliding plate-cover 34 is driven by the cover gear rack 36 to move relative to the positioning slider 31. As a result, the sliding plate-cover 34 is movable to a position above the positioning slider 31 to cover the transfer plate 92 and to prevent foreign objects from falling into the solution recesses 921.

The structure for moving the sliding plate-cover 34 is not limited thereby, as long as the sliding plate-cover 34 is capable of selectively covering the solution recesses 921 of the transfer plate 92.

The injection mechanism 40 is mounted on the base 10 and adjacent to the transfer plate positioning mechanism 30. The injection mechanism 40 has multiple injection heads 41. Each of the injection heads 41 is in fluid communication with a respective one of the solutions 91, and is movable to the receiving area 102 of the base 10. The aforementioned positioning slider 31 is configured to align any one of the solution recesses 921 of the transfer plate 92 to the injection head 41 that is located in the receiving area 102 such that said injection head 41 injects one of the solutions 91 into the corresponding solution recess 921.

In the preferred embodiment, the injection mechanism 40 has a rotary seat 42 rotatably mounted on the base 10. The injection heads 41 are mounted on the rotary seat 42 and disposed apart from each other around a rotating axis of the rotary seat 42. Rotation of the rotary seat 42 moves the injection heads 41 to the receiving area 102 for a given positioning, and then the injection head 41 in the receiving area 102 is aligned to the respective solution recess 921 by controlling the positioning slider 31 to the fine-tune position of the transfer plate 92 in both the longitudinal direction L and the transverse direction T.

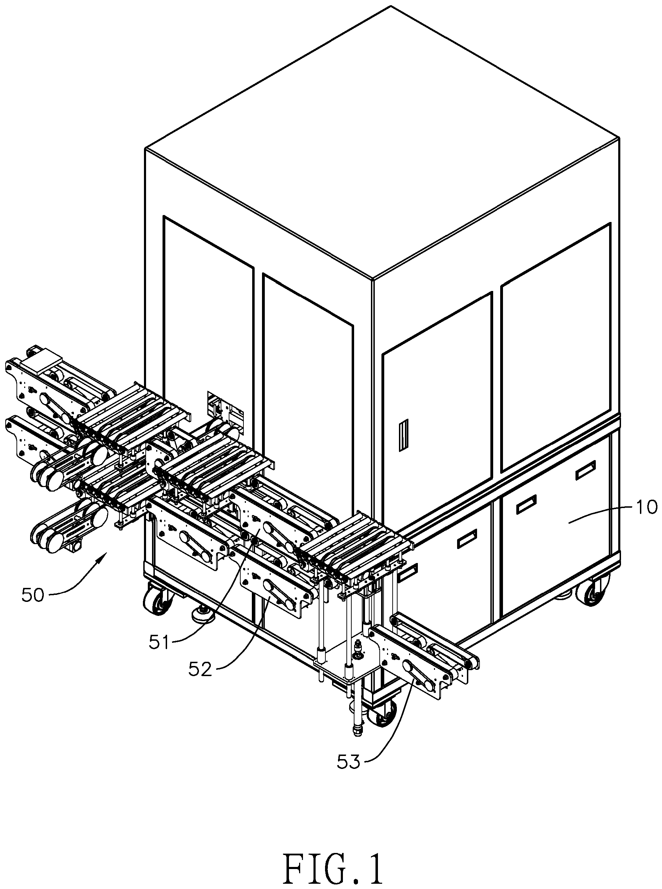

With reference to Figs. 1 and 2, the cell culture plate conveyer 50 is located on a side of the base 10 and configured to deliver the ready-to-process cell culture plate 94 from a cell culture device (not shown in figures) to the base 10. Processed cell culture plate 94 is also returned to the cell culture device by the cell culture plate conveyer 50.

The cell culture plate conveyer 50 has an input conveyor belt 51, an output conveyor belt 52, and a cell culture plate elevator 53. The input conveyor belt 51 is configured to deliver the ready-to-process cell culture plate 94 to the cell culture plate positioning mechanism 60. The output conveyor belt 52 is disposed under the input conveyor belt 51. The input conveyor belt 51 and the output conveyor belt 52 operate in reverse directions. The cell culture plate elevator 53 is mounted to an end of the input conveyor belt 51, and is capable of moving downward to an end of the output conveyor belt 52 to transfer the cell culture plate 94 from the input conveyor belt 51 to the output conveyor belt 52.

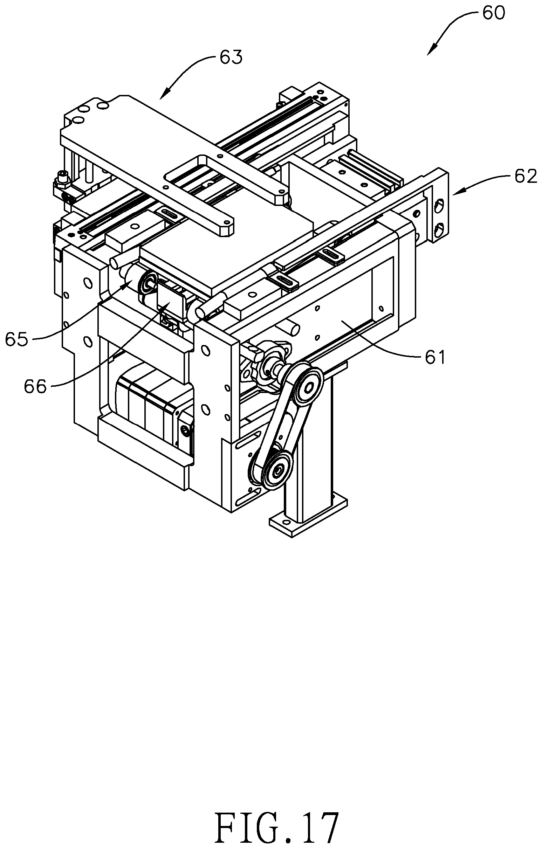

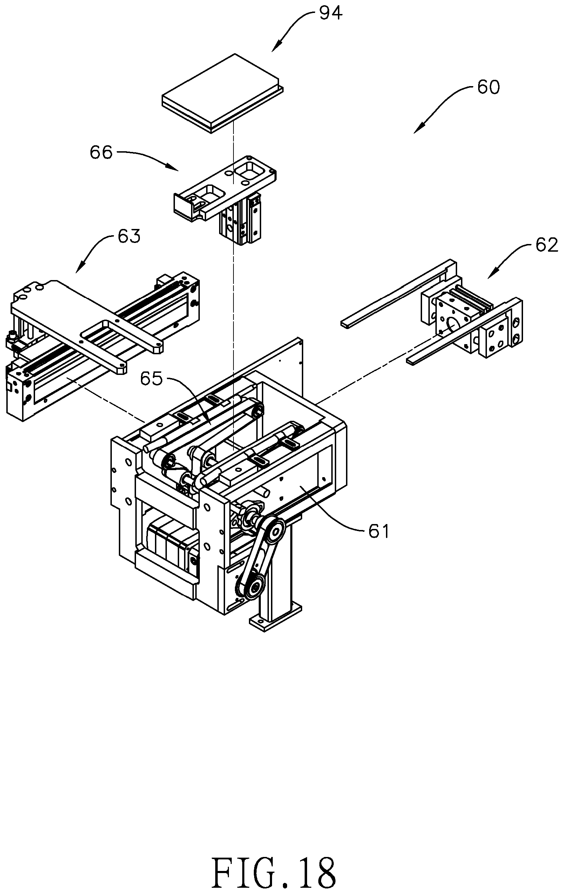

With reference to Figs. 17 and 18, the cell culture plate positioning mechanism 60 is mounted on the base 10 and configured to connect with the cell culture plate 94. The cell culture plate positioning mechanism 60 has a positioning seat 61 and a primary positioning module 62; in the preferred embodiment, the cell culture plate positioning mechanism 60 further has a lid opener 63, multiple suction cups 64, a cell culture plate transfer module 65, and a secondary positioning module 66.

The positioning seat 61 is mounted on the base 10 and configured to accommodate the cell culture plate 94. In the preferred embodiment, the cell culture plate 94 is automatically delivered to the positioning seat 61 by the cell culture plate conveyer 50. Processed cell culture plates 94 are also returned to the cell culture device by the cell culture plate conveyer 50.

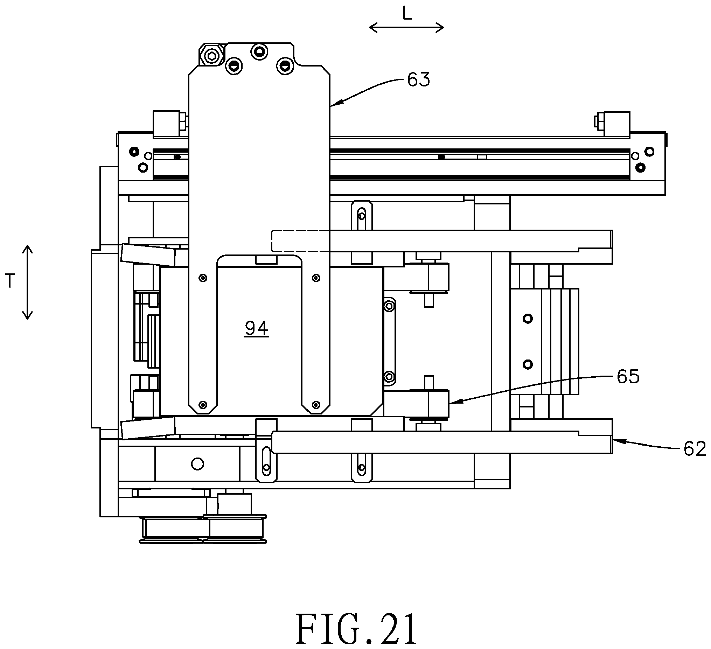

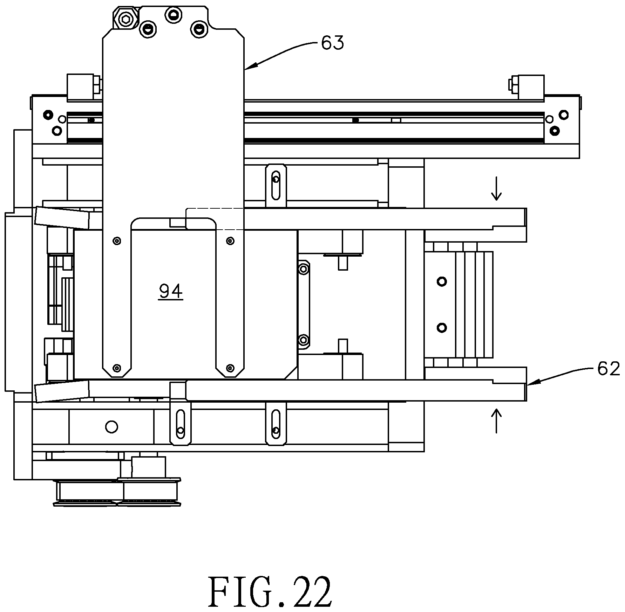

With reference to Figs. 21 and 22, the primary positioning module 62 is mounted on the positioning seat 61 and configured to clamp the two opposite sides of the cell culture plate 94. To be precise, the primary positioning module 62 is a 2-Jaw parallel gripper, and can be controlled to clamp the cell culture plate 94 such that a position of the cell culture plate 94 in the transverse direction T is fixed. The primary positioning module 62 preferably clamps the two opposite sides of the cell culture plate 94 that face toward and move longitudinally away from the transverse direction T.

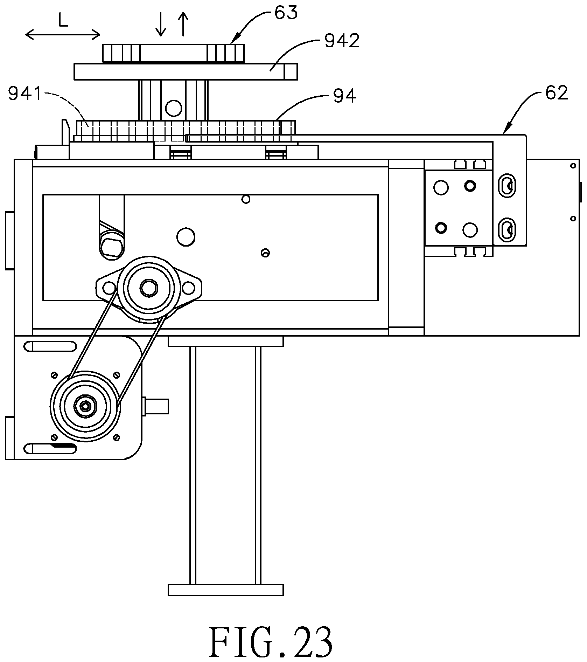

With reference to Figs. 23 and 24, the lid opener 63 is mounted above the positioning seat 61. The lid opener 63 is capable of moving upward and downward, and is capable of moving sideways. The lid opener 63 is movable to a position above the cell culture plate 94. The suction cups 64 are mounted on a bottom surface of the lid opener 63 and are configured to adhere to the upper lid 942 of the cell culture plate 94. In the preferred embodiment, the suction cups 64 are connected to a vacuum generator (not shown in figures) such that the suction cups 64 are adhered to the upper lid 942 by vacuum pressure. In another preferred embodiment, one suction cup 64 is sufficient for adhering to the upper lid 942.

With reference to Figs. 18 to 20, the cell culture plate transfer module 65 is mounted in the positioning seat 61, and is preferably a pair of parallel conveyer belts. The cell culture plate transfer module 65 is capable of moving the cell culture plate 94 rested on the positioning seat 61 toward or away from the cell culture plate conveyer 50.

The secondary positioning module 66 is mounted in the positioning seat 61, is capable of moving upward or downward, and is configured to lift the cell culture plate 94 rested on the positioning seat 61. When the cell culture plate 94 is lifted by the secondary positioning module 66, two ends of the secondary positioning module 66 attach two opposite sides of the cell culture plate 94 facing toward or moving along with the longitudinal direction L to fix a position of the cell culture plate 94 in the longitudinal direction L.

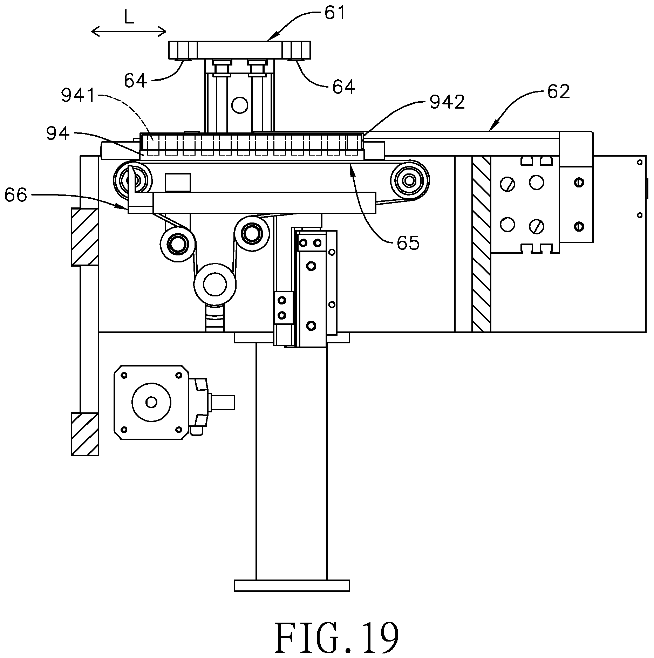

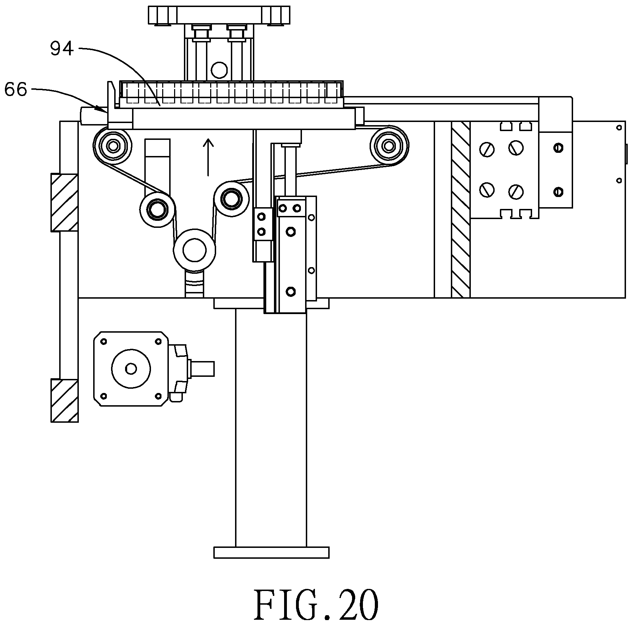

Detailed operating steps of the cell culture plate positioning mechanism 60 are as described below.
1. The cell culture plate conveyer 50 delivers a ready-to-process cell culture plate 94 to the positioning seat 61. The cell culture plate transfer module 65 roughly adjust a position of the cell culture plate 94 on the positioning seat 61.
2. With reference to Figs. 19 and 20, the secondary positioning module 66 moves upward to lift the cell culture plate 94 and fix the position of the cell culture plate 94 in the longitudinal direction L.
3. With reference to Figs. 21 and 22, the primary positioning module 62 clamps the two opposite sides of the cell culture plate 94 that face toward and move longitudinally away from the transverse direction T such that a position of the cell culture plate 94 in the transverse direction T is fixed.
4. With reference to Fig. 23, the lid opener 63 moves downward to adhere to the upper lid 942 with the suction cups 64, and then the lid opener 63 moves upwards to separate the upper lid 942 from the cell culture plate 94.
5. With reference to Figs. 23 and 24, the lid opener 63 moves sideways away from the position above the cell culture plate 94 to uncover partially the wells 941 of the cell culture plate 94.

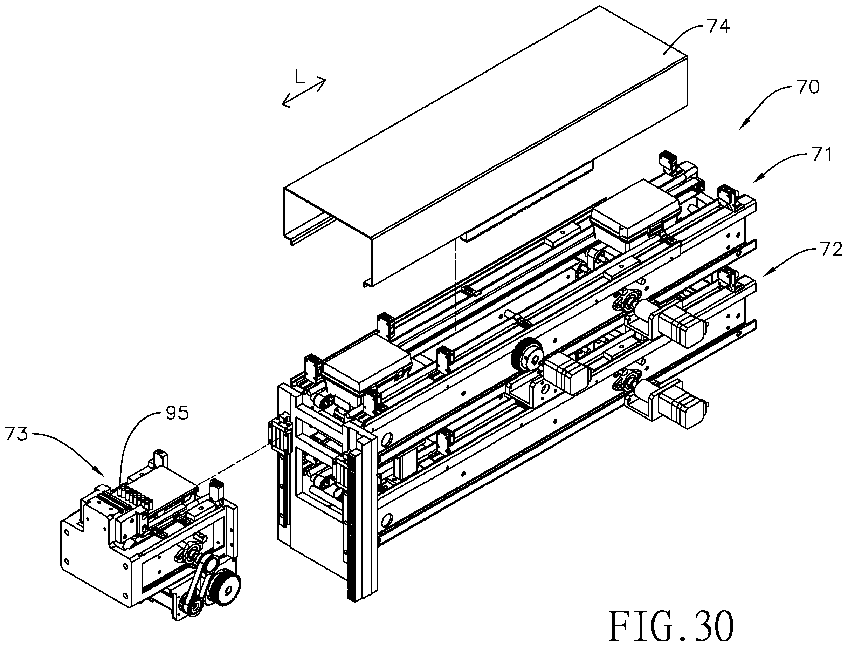

With reference to Figs. 30 to 32, the pipette-tip feeder 70 is mounted on the base 10 and configured to deliver pipette-tips 95 packed in boxes. The pipette-tip feeder 70 has an input conveyer belt 71, an output conveyer belt 72, a tip positioning module 73, a sliding tip cover 74, and at least one UV lamp 75.

The tip positioning module 73 is mounted on an end of the input conveyer belt 71. The output conveyer belt 72 is mounted under the input conveyer belt 71. The output conveyer belt 72 and the input conveyer belt 71 operate in opposite directions. The tip positioning module 73 is capable of moving downward to align with an end of the output conveyer belt 72. The input conveyer belt 71 is configured to deliver unused pipette-tips 95 packed in boxes to the tip positioning module 73, and then the tip positioning module 73 fine-tunes a position of the pipette-tip 95 for other mechanism of the present invention.

The sliding tip cover 74 is movably mounted on the input conveyer belt 71 and is movable to a position above the tip positioning module 73 to cover partially the pipette-tips 95 in use, thereby preventing the pipette-tips 95 unused yet from being contaminated. At least one UV lamp 75 is mounted in an inner surface of the sliding tip cover 74 to maintain the unused pipette-tips 95 in a sterilized condition with ultraviolet irradiation.

With reference to Figs. 25 to 27, the dispensing mechanism 80 is mounted on the base 10. The dispensing mechanism 80 has a dispensing seat 81. In the preferred embodiment, the dispensing mechanism 80 further has a first dispensing linear module 82, a second dispensing linear module 83, a third dispensing linear module 84, a tip ejector 85, a tilt actuator 86, and a pivot axle 87.

The dispensing seat 81 is configured to fix the pipette 93 and configured to control aspirate and discharge operation of the pipette 93. The dispensing seat 81 is movable relative to the base 10 and is movable back and forth between a position above the dispensing area 103 of the base 10 and a position above the cell culture plate positioning mechanism 60. When the dispensing seat 81 is above the dispensing area 103, the dispensing seat 81 is configured to insert the pipette 93 with the at least one pipette-tip 95 into any one of the solution recesses 921 of the transfer plate 92 and execute the aspirate operation of the pipette 93 to draw up liquid inside the solution recess 921. When the dispensing seat 81 is above the cell culture plate positioning mechanism 60, the dispensing seat 81 executes the discharge operation of the pipette 93 to release the solution 91 aspired in the at least one pipette-tip 95 into one of the wells 941 of the cell culture plate 94.

In the preferred embodiment, the dispensing seat 81 moves and inserts the pipette-tip 95 into the wells 941 via the first dispensing linear module 82, the second dispensing linear module 83, and the third dispensing linear module 84. To be specific, the first dispensing linear module 82 is mounted on the base 10 and extends in the transverse direction T. The second dispensing linear module 83 is mounted on the first dispensing linear module 82 and extends in the longitudinal direction L. The third dispensing linear module 84 is mounted on the second dispensing linear module 83 and extends in vertical direction. The dispensing seat 81 is mounted on the third dispensing linear module 84. As a result, the dispensing seat 81 has 3 degrees of freedom via the dispensing linear modules 82, 83, and 84.

Additionally, the dispensing seat 81 in this embodiment is movable to a position above the tip positioning module 73 of the pipette-tip feeder 70, and then the dispensing seat 81 is movable toward the tip positioning module 73 to connect with the new pipette-tips 95. Furthermore, the dispensing seat 81 is pivotally mounted on the base 10. Rotation of the dispensing seat 81 relative to the base 10 is capable of aligning the tip connectors 931 of the pipette 93 with pipette-tips 95 on the tip positioning module 73 such that the centerline of the tip connectors 931 are parallel to the centerline of the pipette-tips 95; the rotation of the dispensing seat 81 relative to the base 10 is also capable of making the centerline of the tip connectors 931 inclined to the centerline of the pipette-tips 95.

With reference to Figs. 25, 32, and 33, to be precise, the dispensing seat 81 is pivotally mounted on a connecting seat 841 of the third dispensing linear module 84 via the pivot axle 87, and the pivot axle 87 is nonparallel to the tip connectors 931. To be more precise, the pivot axle 87 substantially extends in the transverse direction T, and the pivot axle 87 is substantially perpendicular to the tip connectors 931. As a result, the rotation of the dispensing seat 81 is capable of adjusting the centerline of the tip connectors 931 by the vertical line; it means that the rotation of the dispensing seat 81 is also capable of tilting the tip connectors 931 such that the centerline of the tip connectors 931 form an adjustable angle with the vertical line.

In the preferred embodiment, the tilt actuator 86 is a linear actuator mounted on the connecting seat 841. A shaft 861 of the tilt actuator 86 is connected to the dispensing seat 81 to control a rotating angle of the dispensing seat 81 around the pivot axle 87.

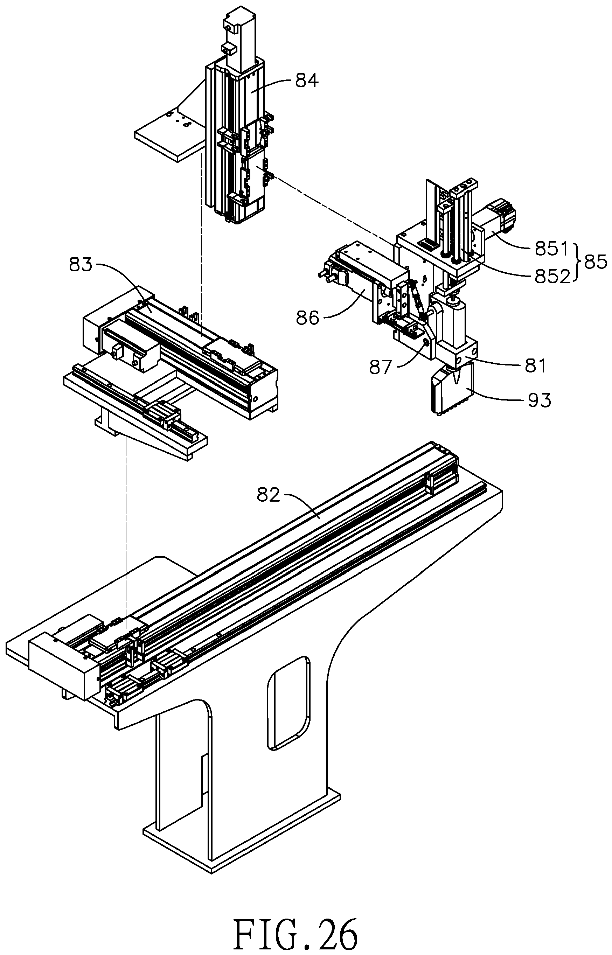

The tip ejector 85 is mounted on the dispensing seat 81 and configured to press against the pipette 93 to remove the pipette-tip 95 from the pipette 93. With reference to Fig. 26, the tip ejector 85 has a motor 851 and a rack 852, and the motor 851 drives the rack 852 to press against a release button (not shown in figures) on the pipette 93 to remove the pipette-tip 95 from the pipette 93.

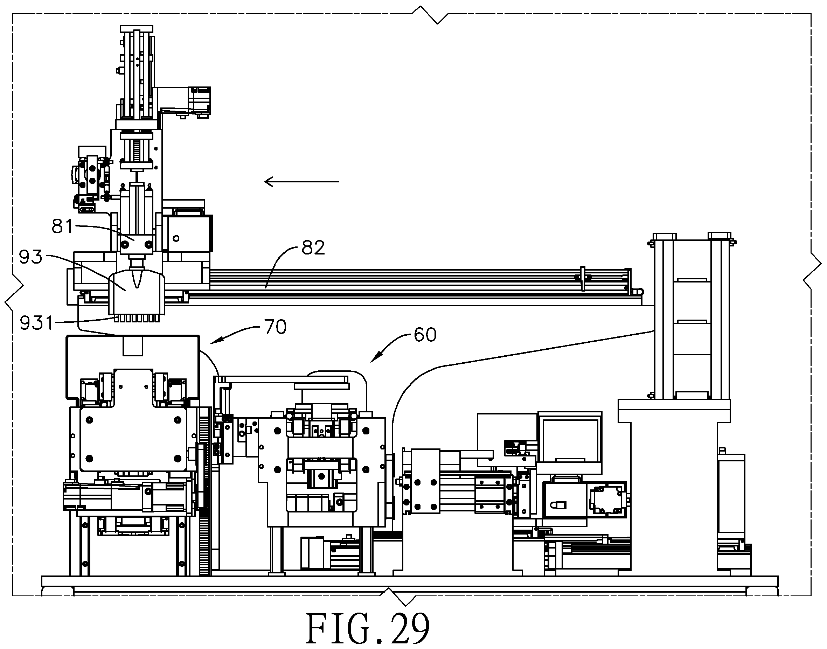

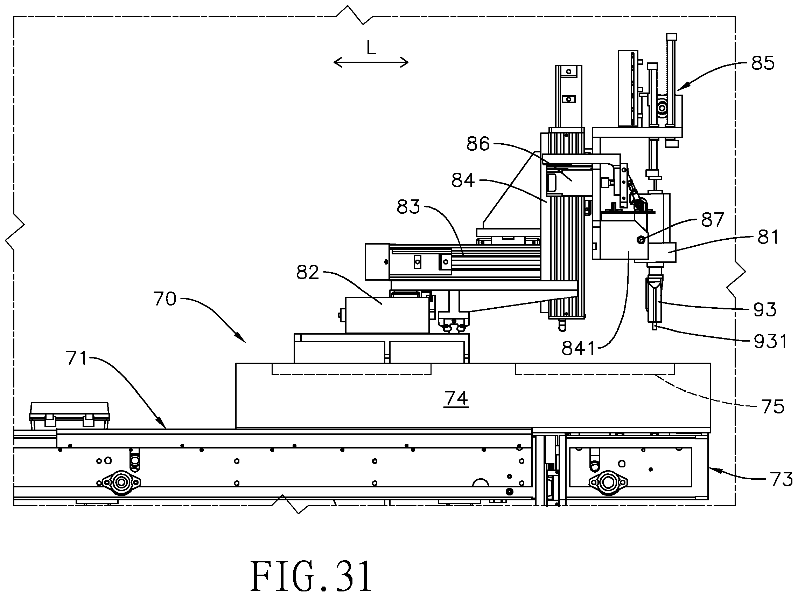

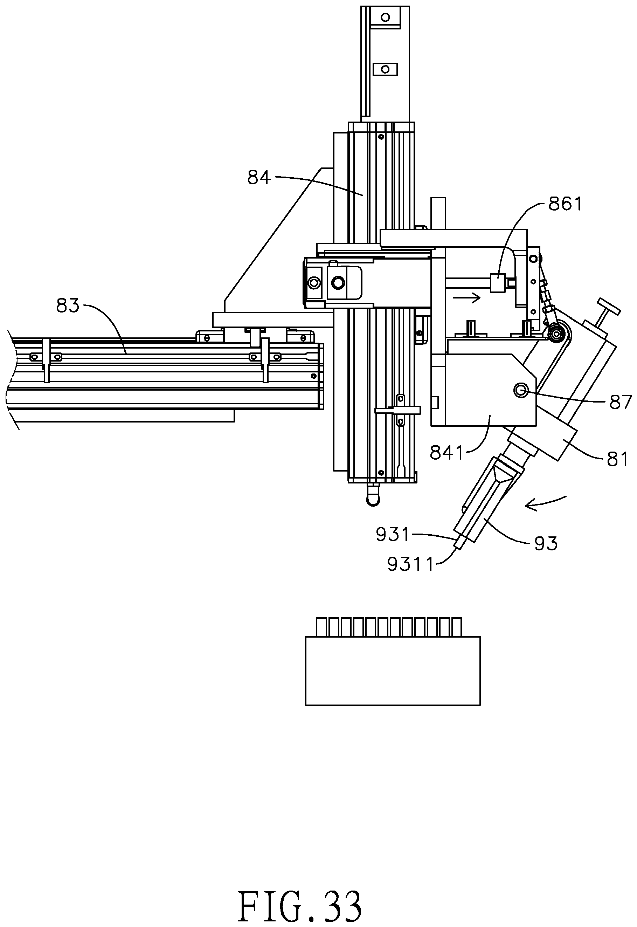

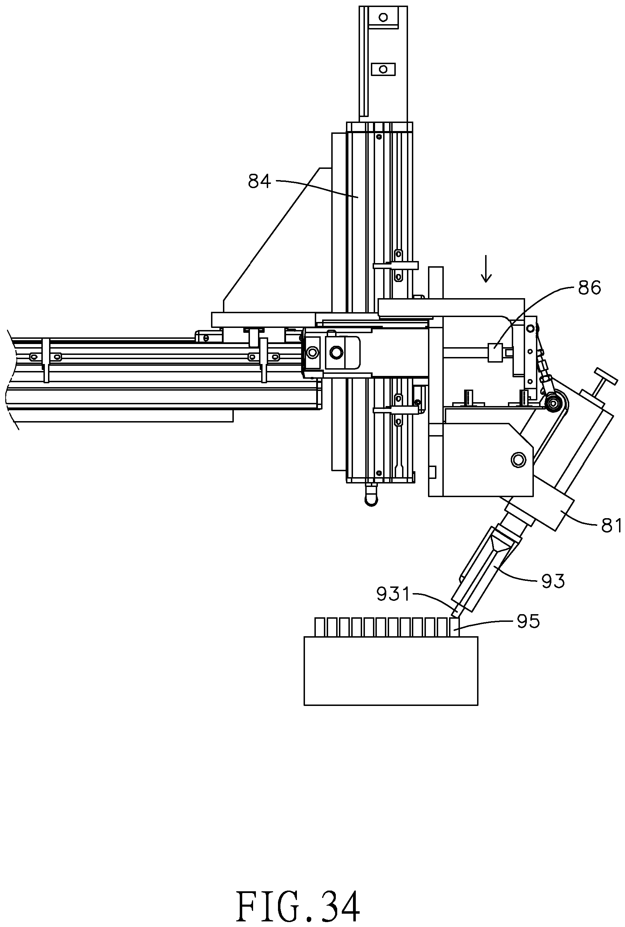

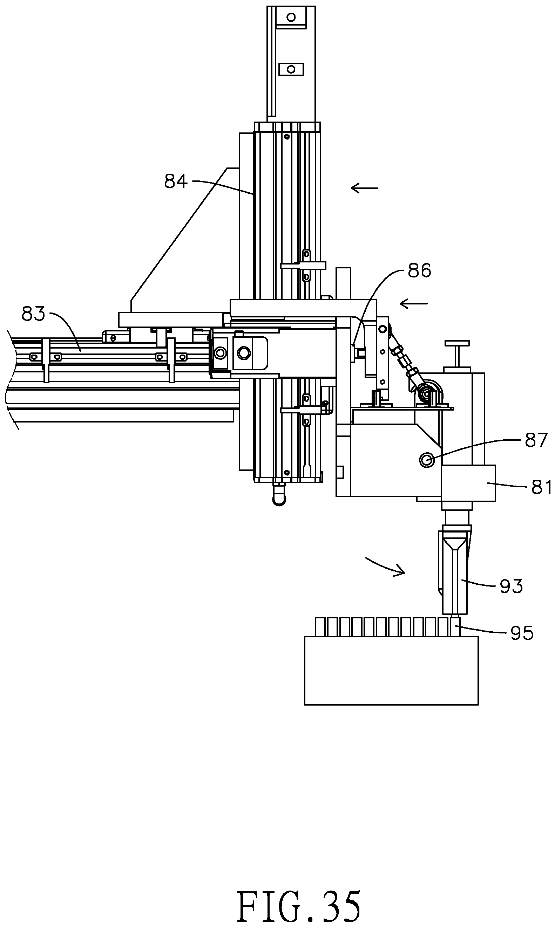

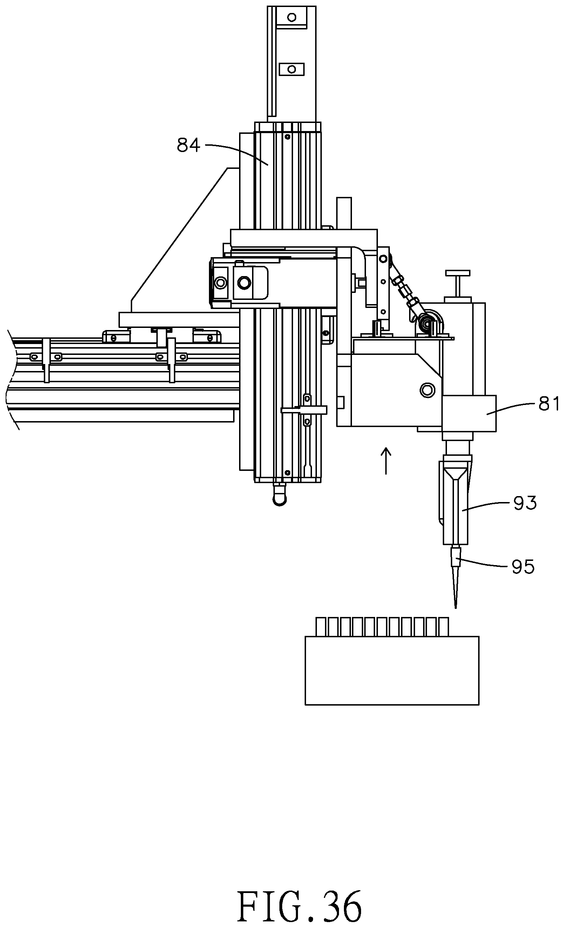

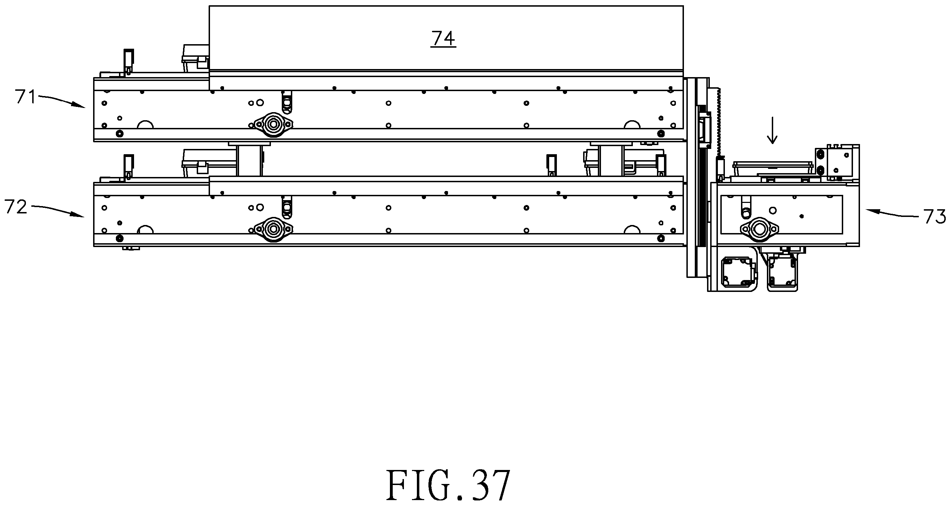

Coordination between the pipette-tip feeder 70 and the dispensing mechanism 80 makes automatic replacement of the pipette-tips 95 possible, and detailed operating steps are as described below.
1. The tip ejector 85 presses against the release button on the pipette 93 to remove the pipette-tips 95 from the pipette 93.
2. With reference to Figs. 29 and 31, the dispensing seat 81 moves to the position above the tip positioning module 73.
3. With reference to Fig. 32, the sliding tip cover 74 moves oppositely away from the tip positioning module 73 to reveal the pipette-tips 95 on the tip positioning module 73.
4. With reference to Fig. 33, the dispensing seat 81 is tilted by the tilt actuator 86 such that the centerline of the tip connectors 931 form an angle with the vertical line.
5. With reference to Figs. 33 and 34, the dispensing seat 81 moves downward to partially insert a distal end 9311 (as shown in Fig. 33) of each of the tip connectors 931 into a respective one of the pipette-tips 95.
6. With reference to Figs. 35 and 36, the tilt actuator 86 gradually straightens the dispensing seat 81 to align the centerline of the tip connectors 931 to match with the vertical line again. In the meantime, the dispensing linear modules 82, 83, and 84 drive the dispensing seat 81 to gradually press the tip connectors 931 against the pipette-tips 95 such that the tip connectors 931 are eventually connected with the pipette-tips 95, and then the dispensing seat 81 moves upward to continue dispensing operation.
7. With reference to Fig. 37, when the pipette-tips 95 delivered in a box have been depleted, the tip positioning module 73 moves downward to align with the end of the output conveyer belt 72 to send away the empty box using the output conveyer belt 72.

The box retriever for the box of pipette-tips 95 is substantially similar to the transfer plate serving mechanism 21 aforementioned. The difference between the transfer plate retriever 22 and the transfer plate serving mechanism 21 is that the transfer plate retriever 22 is configured to recycle used transfer plates 92, and therefore operating procedure of the transfer plate retriever 22 is in a reverse order compared with the transfer plate serving mechanism 21. That is, the transfer plate retriever 22 detaches the used transfer plates 92 with the transfer plate positioning mechanism 30, and then stacks the used transfer plates 92 in a bottom-up way.

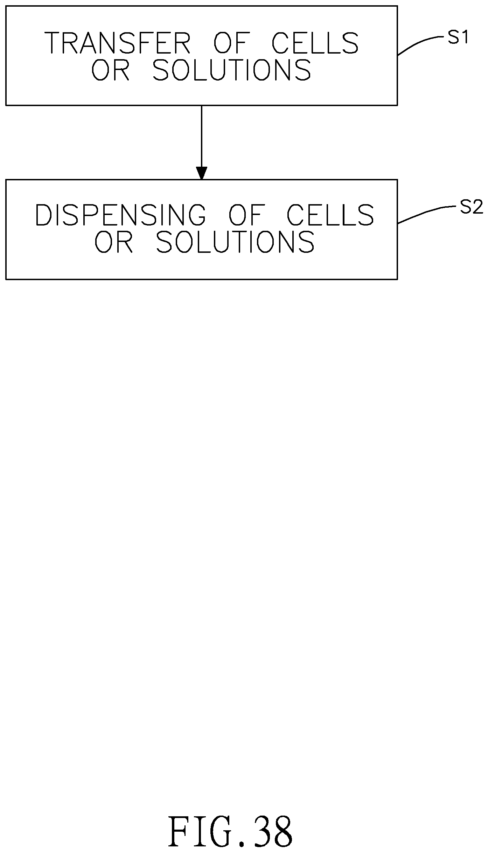

With reference to Fig. 38, a cell and medicament dispensing method for drug screening in accordance with the present invention comprises the following steps: the first step (S1) is transfer of cells or solutions; the second step (S2) is dispensing of cells or solutions. The cell and medicament dispensing method is performed by the aforementioned cell and medicament dispensing device, but not limited thereto.

The first step (S1) is transfer of cells or solutions. With reference to Figs. 10 to 15, the plate feeder 213 of the transfer plate serving mechanism 21 moves the transfer plate 92 to the transfer plate entrance area 101 on the base 10. The transfer plate 92 has multiple solution recesses 921 as aforementioned. The positioning slider 31 of the transfer plate positioning mechanism 30 moves to the transfer plate entrance area 101 and connects with the transfer plate 92. The positioning slider 31 moves the transfer plate 92 to the receiving area 102 of the base 10.

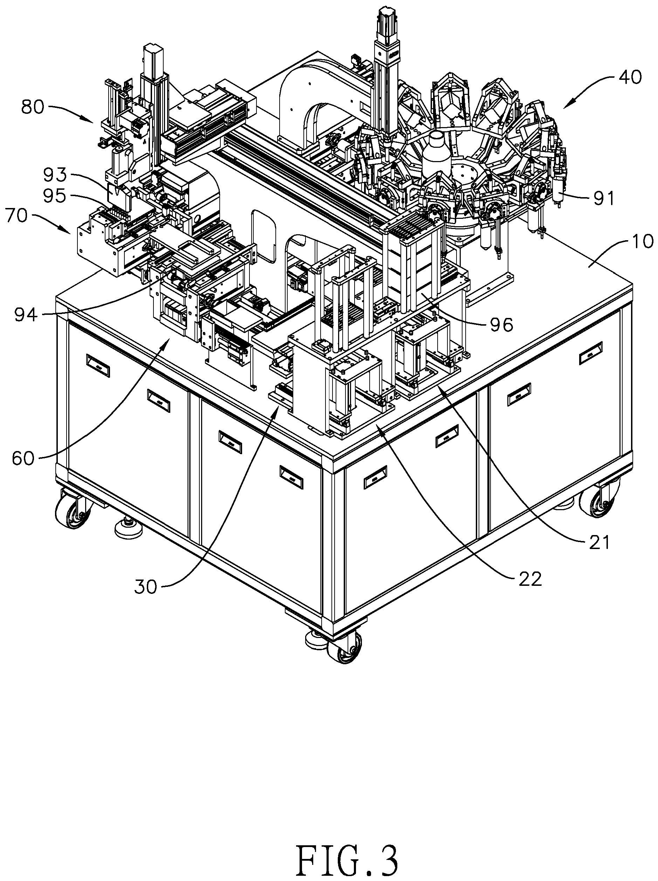

With reference to Figs. 3, 6, and 15, the injection mechanism 40 injects multiple solutions 91 into the solution recesses 921 of the transfer plate 92. Each of the solution recesses 921 may be injected with one single solution 91, or may be injected with multiple kinds of the solutions 91. Each of the solutions 91 comprises one single type of medicament or cells. In the preferred embodiment, the sliding plate-cover 34 partially covers the openings of the solution recesses 921 to prevent foreign objects from falling into the unused part of solution recesses 921.

With reference to Figs. 8 and 9, multiple spare transfer plates 96 can be stacked on the transfer plate serving mechanism 21. After the plate feeder 213 moves the transfer plate 92 to the transfer plate entrance area 101 of the base 10, the transfer plate serving mechanism 21 moves the bottommost spare transfer plate 96 to the plate feeder 213. By moving the bottommost spare transfer plate 96 first, risk of foreign objects falling into the spare transfer plate 96 is minimized.

In the preferred embodiment, the plate-locking clamps 212 first move away from each other, allowing the spare transfer plate 96 to drop down. In the meantime, the plate lifting actuator 214 moves upward and protrudes through the plate lifting opening 2131 such that only the bottommost spare transfer plate 96 is located under the plate dropping opening 2111, keeping the other spare transfer plates 96 still above the plate dropping opening 2111. Then, the plate-locking clamps 212 move toward each other to prevent the other spare transfer plates 96 from dropping through the plate dropping opening 2111, and then the plate lifting actuator 214 moves downward to its original position such that the bottommost spare transfer plate 96 can stay and fix on the plate feeder 213.

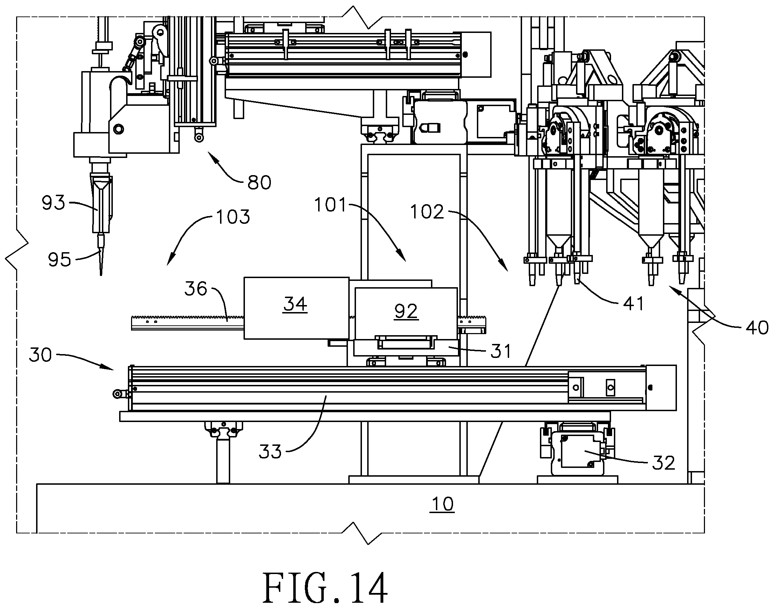

The second step (S2) is dispensing of cells or solutions. With reference to Figs. 14 to 16, the positioning slider 31 moves the transfer plate 92 to the dispensing area 103 on the base 10. The cell culture plate 94 has multiple wells 941 as aforementioned. The dispensing mechanism 80 moves the pipette 93 back and forth between the position above the dispensing area 103 and the position above the cell culture plate 94 to dispense liquid in each of the solution recesses 921 of the transfer plate 92 into the wells 941 of the cell culture plate 94.

In the preferred embodiment, the sliding plate-cover 34 moves away from the transfer plate 92 to uncover one of the openings of the solution recesses 921. After the pipette 93 dispenses liquid in said one of the openings of the solution recesses 921, the sliding plate-cover 34 moves back toward the transfer plate 92 to cover fully the openings of the solution recesses 921 again.

In summary, by coordination among the transfer plate serving mechanism 21, the transfer plate positioning mechanism 30, and the injection mechanism 40, liquids and cells for drug screening are automatically transferred to the solution recesses 921 of the transfer plate 92. Afterwards, liquid in each of the solution recesses 921 is automatically dispensed into the wells 941 of the cell culture plate 94 by coordination between the transfer plate positioning mechanism 30 and the dispensing mechanism 80. As a result, the process of drug screening is automated to reduce labor and improve quality significantly.

## Claims

1. A cell and medicament dispensing device for drug screening, **characterized in that** the cell and medicament dispensing device is configured to inject multiple solutions (91) into multiple solution recesses (921) of a transfer plate (92); then, liquid inside each of the solution recesses (921) dispensed into multiple wells (941) of a cell culture plate (94) with a pipette (93); at least one pipette-tip (95) detachably connected to a bottom of the pipette (93); the cell and medicament dispensing device comprising:
a base (10) having a transfer plate entrance area (101), a receiving area (102), and a dispensing area (103) thereon;
a transfer plate serving mechanism (21) mounted on the base (10) and being adjacent to the transfer plate entrance area (101); the transfer plate serving mechanism (21) configured to accommodate the transfer plate (92) and being capable of moving the transfer plate (92) to the transfer plate entrance area (101) of the base (10), the transfer plate serving mechanism (21) having
a plate stacking bracket (211) forming a stacking space; the stacking space configured to accommodate multiple spare transfer plates (96) that are stacked vertically;
a plate dropping opening (2111) formed in a bottom of the plate stacking bracket (211); the spare transfer plates (96) being capable of dropping through the plate dropping opening (2111);
two plate-locking clamps (212); each of the plate-locking clamps (212) mounted on a respective side of two opposite sides of the plate dropping opening (2111); the two plate-locking clamps (212) being capable of moving toward each other to prevent the spare transfer plates (96) from passing through the plate dropping opening (2111);
a plate feeder (213) mounted under the plate dropping opening (2111) and configured to receive the transfer plates (92) dropped through the plate dropping opening (2111); the plate feeder (213) being movable to the transfer plate entrance area (101) of the base (10); the plate feeder (213) having:
a plate lifting opening (2131) formed through two opposite sides of the plate feeder (213); and
a plate lifting actuator (214) mounted under the plate lifting opening (2131) and being capable of moving upward to protrude through the plate lifting opening (2131);
a transfer plate positioning mechanism (30) mounted on the base (10) and being adjacent to the transfer plate serving mechanism (21); the transfer plate positioning mechanism (30) having:
a positioning slider (31) configured to connect with the transfer plate (92); the positioning slider (31) movably mounted on the base (10) and selectively corresponding in position to the transfer plate entrance area (101), the receiving area (102), or the dispensing area (103), wherein the plate feeder (213) is configured to move to a position above the positioning slider (31) to transfer the transfer plate (92) to the positioning slider (31);
an injection mechanism (40) mounted on the base (10) and being adjacent to the transfer plate positioning mechanism (30); the injection mechanism (40) having:
multiple injection heads (41); each of the injection heads (41) being in fluid communication with a respective one of the solutions (91) and being movable to the receiving area (102) of the base (10); wherein the positioning slider (31) is configured to align any one of the solution recesses (921) of the transfer plate (92) to the injection head (41) located in the receiving area (102) such that said injection head (41) injects one of the solutions (91) into a corresponding one of the solution recesses(921);
a cell culture plate positioning mechanism (60) mounted on the base (10) and configured to connect with the cell culture plate (94); the cell culture plate positioning mechanism (60) having:
a positioning seat (61) mounted on the base (10) and configured to accommodate the cell culture plate (94); and
a primary positioning module (62) mounted on the positioning seat (61) and configured to clamp the cell culture plate (94); and
a dispensing mechanism (80) mounted on the base (10) and having:
a dispensing seat (81) configured to fix the pipette (93) and configured to control aspirate and discharge operation of the pipette (93); the dispensing seat (81) being movable relative to the base (10) and being movable back and forth between a position above the dispensing area (103) of the base (10) and a position above the cell culture plate positioning mechanism (60);
wherein the dispensing seat (81) is configured to move to a position above the dispensing area (103) to insert the at least one pipette-tip (95) of the pipette (93) into any one of the solution recesses (921) of the transfer plate (92) and execute the aspirate operation of the pipette (93); and
wherein the dispensing seat (81) is configured to move to a position above the cell culture plate positioning mechanism (60)and execute the discharge operation of the pipette (93) to release the solution (91) aspired in the at least one pipette-tip (95) into one of the wells (941) of the cell culture plate (94).

2. The cell and medicament dispensing device as claimed in claim 1 further comprising:
a pipette-tip feeder (70) mounted on the base (10) and having
an input conveyer belt (71); and
a tip positioning module (73) mounted on an end of the input conveyer belt (71);
the dispensing seat (81) of the dispensing mechanism (80) being movable to a position above the tip positioning module (73) of the pipette-tip feeder (70) and moveable toward the tip positioning module (73).

3. The cell and medicament dispensing device as claimed in claim 2, wherein
the dispensing seat (81) of the dispensing mechanism (80) is pivotal around a pivot axle (87); the pivot axle (87) is nonparallel to the vertical line;
the dispensing mechanism (80) further has
a tilt actuator (86) connected to the dispensing seat (81) and controlling a rotating angle of the dispensing seat (81) around the pivot axle (87).

4. The cell and medicament dispensing device as claimed in claim 2, wherein
the pipette-tip feeder (70) further has:
an output conveyer belt (72) mounted under the input conveyer belt (71); the output conveyer belt (72) and the input conveyer belt (71) operating in opposite directions;
the tip positioning module (73) is capable of moving downward to align with an end of the output conveyer belt (72).

5. The cell and medicament dispensing device as claimed in any one of claims 1 to 4, wherein
the cell culture plate (94) has an upper lid (942) detachably covering the wells (941); and
the cell culture plate positioning mechanism (60) further has
a lid opener (63) mounted above the positioning seat (61); the lid opener (63) being capable of moving upward and downward, and capable of moving sideways; the lid opener (63) being movable to a position above the cell culture plate (94); and
at least one suction cup (64) mounted on a bottom surface of the lid opener (63) and configured to adhere to the upper lid (942) of the cell culture plate (94).

6. The cell and medicament dispensing device as claimed in any one of claims 1 to 4, wherein
the positioning slider (31) of the transfer plate positioning mechanism (30) is capable of moving upward and downward; and
when the positioning slider (31) corresponds in position to the transfer plate entrance area (101), the plate feeder (213) of the transfer plate serving mechanism (21) is movable to a position above the positioning slider (31), and the positioning slider (31) is capable of moving upward to protrude through the plate lifting opening (2131).

7. The cell and medicament dispensing device as claimed in any one of claims 1 to 4, wherein the transfer plate positioning mechanism (30) has
a sliding plate-cover (34) movably mounted on the positioning slider (31) and being movable to a position above the positioning slider (31) to cover openings of the solution recesses (921) of the transfer plate (92).

8. A cell and medicament dispensing method for drug screening, **characterized in that** the cell and medicament dispensing method comprises steps as follows:
(a) transfer of cells or solutions (91), wherein a transfer plate serving mechanism (21) has a plate stacking bracket (211), a plate dropping opening (2111), two plate-locking clamps (212), a plate feeder (213), and a plate lifting actuator (214); the plate stacking bracket (211) forms a stacking space; the stacking space is configured to accommodate multiple spare transfer plates (96) that are stacked vertically; the plate dropping opening (2111) is formed in a bottom of the plate stacking bracket (211); the spare transfer plates (96) are capable of dropping through the plate dropping opening (2111); the two plate-locking clamps (212) are each mounted on a respective side of two opposite sides of the plate dropping opening (2111); the two plate-locking clamps (212) are capable of moving toward each other to prevent the spare transfer plates (96) from passing through the plate dropping opening (2111); the plate feeder (213) is mounted under the plate dropping opening (2111) and is configured to receive a transfer plate (92) dropped through the plate dropping opening (2111); the plate feeder (213) is movable to the transfer plate entrance area (101) of the base (10) and has a plate lifting opening (2131) formed through two opposite sides of the plate feeder (213); the plate lifting actuator (214) is mounted under the plate lifting opening (2131) and is capable of moving upward to protrude through the plate lifting opening (2131); the plate feeder (213) of the transfer plate serving mechanism (21) moves the transfer plate (92) to a transfer plate entrance area (101) on a base (10); the transfer plate (92) has multiple solution recesses (921) formed thereon; a positioning slider (31) of a transfer plate positioning mechanism (30) moves to the transfer plate entrance area (101) and connects with the transfer plate (92) located in the transfer plate entrance area (101); the plate feeder (213) of the transfer plate serving mechanism (21) moves to the position above the positioning slider (31) to transfer the transfer plate (92) to the positioning slider (31); the positioning slider (31) moves the transfer plate (92) to a receiving area (102) on the base (10); an injection mechanism (40) injects multiple solutions (91) into the solution recesses (921) of the transfer plate (92); and
(b) dispensing of cells or solutions (91), wherein the positioning slider (31) moves the transfer plate (92) to a dispensing area (103) on the base (10); a cell culture plate (94) has multiple wells (941); a dispensing mechanism (80) moves a pipette (93) back and forth between a position above the dispensing area (103) on the base (10) and a position above the cell culture plate (94) on the positioning slider (31) to dispense liquid in each of the solution recesses (921) into the wells (941) of the cell culture plate (94).

9. The cell and medicament dispensing method as claimed in claim 8, wherein in the step (a), after the plate feeder (213) moves the transfer plate (92) to the transfer plate entrance area (101) on the base (10), the transfer plate serving mechanism (21) moves the bottommost spare transfer plate (96) to the plate feeder (213).

10. The cell and medicament dispensing method as claimed in claim 8 or 9, wherein
in the step (a), after the injection mechanism (40) injects solutions (91) into the solution recesses (921) of the transfer plate (92), a sliding plate-cover (34) covers openings of the solution recesses (921) of the transfer plate (92); and
in the step (b), the sliding plate-cover (34) moves away from the transfer plate (92) to uncover one of the solution recesses (921) before the pipette (93) dispenses liquid in said one of the solution recesses (921); after the pipette (93) dispenses liquid in said one of the solution recesses (921), the sliding plate-cover (34) moves back toward the transfer plate to cover fully the openings of the solution recesses (921).

## Patentansprüche

1. - Zell- und Medikamentabgabevorrichtung für Arneimittel-Screening, **dadurch gekennzeichnet, dass** die Zell- und Medikamentabgabevorrichtung konfiguriert ist, um mehrere Lösungen (91) in mehrere Lösungsaussparungen (921) einer Transferplatte (92) zu injizieren; dann mit einer Pipette (93) Flüssigkeit innerhalb jeder der Lösungsaussparungen (921) in mehrere Wells (941) einer Zellkulturplatte (94) abzugeben; wobei mindestens eine Pipettenspitze (95) abnehmbar mit einem Boden der Pipette (93) verbunden ist; die Zell- und Medikamentabgabevorrichtung umfassend:
eine Basis (10), die einen Transferplatteneingangsbereich (101), einen Aufnahmebereich (102) und einen Abgabebereich (103) darauf aufweist;
einen Transferplattenzufuhrmechanismus (21), der an der Basis (10) montiert ist und angrenzend an den Transferplatteneingangsbereich (101) ist; wobei der Transferplattenzufuhrmechanismus (21) konfiguriert ist, um die Transferplatte (92) aufzunehmen und in der Lage ist, die Transferplatte (92) zu dem Transferplatteneingangsbereich (101) der Basis (10) zu bewegen, wobei der Transferplattenzufuhrmechanismus (21) Folgendes aufweist
eine Plattenstapelhalterung (211), die einen Stapelraum bildet; wobei der Stapelraum ist konfiguriert, um mehrere Ersatztransferplatten (96) aufzunehmen, die vertikal gestapelt sind;
eine Plattenfallöffnung (2111), die in einem Boden der Plattenstapelhalterung (211) gebildet ist; wobei die Ersatztransferplatten (96) in der Lage sind, durch die Plattenfallöffnung (2111) zu fallen;
zwei Plattenverriegelungsklammern (212); wobei jede der Plattenverriegelungsklammern (212) auf einer jeweiligen Seite von zwei gegenüberliegenden Seiten der Plattenfallöffnung (2111) montiert ist; wobei die zwei Plattenverriegelungsklammern (212) in der Lage sind, sich aufeinander zu zu bewegen, um zu verhindern, dass die Ersatztransferplatten (96) durch die Plattenfallöffnung (2111) hindurchgehen;
einen Plattenzuführer (213), der unter der Plattenfallöffnung (2111) montiert und konfiguriert ist, um die durch die Plattenfallöffnung (2111) gefallenen Transferplatten (92) aufzunehmen; wobei der Plattenzuführer (213) zu dem Transferplatteneingangsbereich (101) der Basis (10) bewegbar ist; wobei der Plattenzuführer (213) Folgendes aufweist:
eine Plattenhebeöffnung (2131), die durch zwei gegenüberliegende Seiten des Plattenzuführers (213) gebildet ist; und
einen Plattenhebeaktuator (214), der unter der Plattenhebeöffnung (2131) montiert und in der Lage ist, sich nach oben zu bewegen, um durch die Plattenhebeöffnung (2131) hervorzustehen;
einen Transferplattenpositionierungsmechanismus (30), der auf der Basis (10) montiert ist und angrenzend an den Transferplattenzufuhrmechanismus (21) ist; wobei der Transferplattenpositionierungsmechanismus (30) Folgendes aufweist:
einen Positionierungsschieber (31), der konfiguriert ist, um mit der Transferplatte (92) verbunden zu werden; wobei der Positionierungsschieber (31) bewegbar an der Basis (10) montiert ist und in seiner Position wahlweise dem Transferplatteneingangsbereich (101), dem Aufnahmebereich (102) oder dem Abgabebereich (103) entspricht, wobei der Plattenzuführer (213) konfiguriert ist, um sich in eine Position oberhalb des Positionierungsschiebers (31) zu bewegen, um die Transferplatte (92) an den Positionierungsschieber (31) zu übergeben;
einen Injektionsmechanismus (40), der auf der Basis (10) montiert ist und angrenzend an den Transferplattenpositionierungsmechanismus (30) ist; wobei der Injektionsmechanismus (40) Folgendes aufweist:
mehrere Injektionsköpfe (41); wobei jeder der Injektionsköpfe (41) in Fluidverbindung mit einer jeweiligen der Lösungen (91) ist und zu dem Aufnahmebereich (102) der Basis (10) bewegbar ist; wobei der Positionierungsschieber (31) konfiguriert ist, um eine der Lösungsaussparungen (921) der Transferplatte (92) auf den in dem Aufnahmebereich (102) befindlichen Injektionskopf (41) auszurichten, sodass der Injektionskopf (41) eine der Lösungen (91) in eine entsprechende der Lösungsaussparungen (921) injiziert;
einen Zellkulturplatten-Positionierungsmechanismus (60), der an der Basis (10) montiert und konfiguriert ist, um mit der Zellkulturplatte (94) verbunden zu werden; wobei der Zellkulturplatten-Positionierungsmechanismus (60) Folgendes aufweist:
einen Positionierungssitz (61), der an der Basis (10) montiert und konfiguriert ist, um die Zellkulturplatte (94) unterzubringen; und
ein primäres Positionierungsmodul (62), das auf dem Positionierungssitz (61) montiert und konfiguriert ist, um die Zellkulturplatte (94) einzuspannen; und
einen Abgabemechanismus (80), der an der Basis (10) montiert ist und Folgendes aufweist:
einen Abgabesitz (81), der konfiguriert ist, um die Pipette (93) zu fixieren, und der konfiguriert ist, um einen Ansaug- und Entleerungsvorgang der Pipette (93) zu steuern; wobei der Abgabesitz (81) in Bezug auf die Basis (10) bewegbar ist und zwischen einer Position oberhalb des Abgabebereichs (103) der Basis (10) und einer Position oberhalb des Zellkulturplatten-Positionierungsmechanismus (60) hin und her bewegbar ist;
wobei der Abgabesitz (81) konfiguriert ist, um sich zu einer Position oberhalb des Abgabebereichs (103) zu bewegen, um die mindestens eine Pipettenspitze (95) der Pipette (93) in eine der Lösungsaussparungen (921) der Transferplatte (92) einzuführen und den Ansaugvorgang der Pipette (93) auszuführen; und
wobei der Abgabesitz (81) konfiguriert ist, um sich in eine Position oberhalb des Zellkulturplatten-Positionierungsmechanismus (60) zu bewegen und den Abgabevorgang der Pipette (93) auszuführen, um die in der mindestens einen Pipettenspitze (95) angesaugte Lösung (91) in eine der Wells (941) der Zellkulturplatte (94) abzugeben.

2. - Zell- und Medikamentabgabevorrichtung nach Anspruch 1, ferner umfassend:
einen Pipettenspitzenzuführer (70), der an der Basis (10) montiert ist und Folgendes aufweist
ein Eingangsförderband (71); und
ein Spitzenpositionierungsmodul (73), das an einem Ende des Eingangsförderbands (71) montiert ist;
wobei der Abgabesitz (81) des Abgabemechanismus (80) in eine Position oberhalb des Spitzenpositionierungsmoduls (73) der Pipettenspitzenzuführung (70) bewegbar ist und in Richtung des Spitzenpositionierungsmoduls (73) bewegbar ist.

3. - Zell- und Medikamentabgabevorrichtung nach Anspruch 2, wobei
der Abgabesitz (81) des Abgabemechanismus (80) um eine Schwenkachse (87) schwenkbar ist; die Schwenkachse (87) nicht parallel zu der vertikalen Linie ist;
der Abgabemechanismus (80) ferner Folgendes aufweist
einen Neigungsaktuator (86), der mit dem Abgabesitz (81) verbunden ist und einen Drehwinkel des Abgabesitzes (81) um die Schwenkachse (87) steuert.

4. - Zell- und Medikamentabgabevorrichtung nach Anspruch 2, wobei
die Pipettenspitzenzuführung (70) ferner Folgendes aufweist:
ein Ausgangsförderband (72), das unter dem Eingangsförderband (71) montiert ist; wobei das Ausgangsförderband (72) und das Eingangsförderband (71) in entgegengesetzten Richtungen betrieben werden;
wobei das Spitzenpositionierungsmodul (73) in der Lage ist, sich nach unten zu bewegen, um sich mit einem Ende des Ausgangsförderbands (72) auszurichten.

5. - Zell- und Medikamentabgabevorrichtung nach einem der Ansprüche 1 bis 4, wobei
die Zellkulturplatte (94) einen oberen Deckel (942) aufweist, der abnehmbar die Wells (941) abdeckt; und
der Zellkulturplatten-Positionierungsmechanismus (60) ferner Folgendes aufweist
einen Deckelöffner (63), der oberhalb des Positionierungssitzes (61) montiert ist; wobei der Deckelöffner (63) in der Lage ist, sich nach oben und nach unten zu bewegen, und in der Lage ist, sich seitwärts zu bewegen; wobei der Deckelöffner (63) in eine Position oberhalb der Zellkulturplatte (94) bewegbar ist; und
mindestens einen Saugnapf (64), der an einer Unterseite des Deckelöffners (63) montiert und konfiguriert ist, um an dem oberen Deckel (942) der Zellkulturplatte (94) zu haften.

6. - Zell- und Medikamentabgabevorrichtung nach einem der Ansprüche 1 bis 4, wobei
der Positionierungsschieber (31) des Transferplattenpositioniermechanismus (30) in der Lage ist, sich aufwärts und abwärts zu bewegen; und
wenn der Positionierungsschieber (31) in seiner Position dem Transferplatteneingangsbereich (101) entspricht, der Plattenzuführer (213) des Transferplattenzufuhrmechanismus (21) zu einer Position oberhalb des Positionierungsschiebers (31) bewegbar ist und der Positionierungsschieber (31) in der Lage ist, sich nach oben zu bewegen, um durch die Plattenhebeöffnung (2131) hervorzustehen.

7. - Zell- und Medikamentabgabevorrichtung nach einem der Ansprüche 1 bis 4, wobei der Transferplattenpositionierungsmechanismus (30) Folgendes aufweist
eine gleitende Plattenabdeckung (34), die bewegbar auf dem Positionierungsschieber (31) montiert ist und zu einer Position oberhalb des Positionierungsschiebers (31) bewegbar ist, um Öffnungen der Lösungsaussparungen (921) der Transferplatte (92) abzudecken.

8. - Zell- und Medikamentenabgabeverfahren für Arzneimittel-Screening, **dadurch gekennzeichnet, dass** das Zell- und Medikamentenabgabeverfahren Schritte wie folgt umfasst:
(a) Übertragen von Zellen oder Lösungen (91), wobei ein Transferplattenzufuhrmechanismus (21) eine Plattenstapelhalterung (211), eine Plattenfallöffnung (2111), zwei Plattenverriegelungsklammern (212), einen Plattenzuführer (213) und einen Plattenhebeaktuator (214) aufweist; die Plattenstapelhalterung (211) einen Stapelraum bildet; der Stapelraum konfiguriert ist, um mehrere Ersatztransferplatten (96) aufzunehmen, die vertikal gestapelt sind; die Plattenfallöffnung (2111) in einem Boden der Plattenstapelhalterung (211) gebildet ist; die Ersatztransferplatten (96) in der Lage sind, durch die Plattenfallöffnung (2111) zu fallen; die zwei Plattenverriegelungsklammern (212) jeweils auf einer Seite von zwei gegenüberliegenden Seiten der Plattenfallöffnung (2111) montiert sind; die zwei Plattenverriegelungsklammern (212) in der Lage sind, sich aufeinander zu zu bewegen, um zu verhindern, dass die Ersatztransferplatten (96) durch die Plattenfallöffnung (2111) hindurchgehen; der Plattenzuführer (213) unter der Plattenfallöffnung (2111) montiert ist und konfiguriert ist, um eine durch die Plattenfallöffnung (2111) gefallene Transferplatte (92) aufzunehmen; der Plattenzuführer (213) zu dem Transferplatteneingangsbereich (101) der Basis (10) bewegbar ist und eine Plattenhebeöffnung (2131) aufweist, die durch zwei gegenüberliegende Seiten des Plattenzuführers (213) gebildet ist; der Plattenhebeaktuator (214) unter der Plattenhebeöffnung (2131) montiert und in der Lage ist, sich nach oben zu bewegen, um durch die Plattenhebeöffnung (2131) hervorzustehen; die Plattenzuführer (213) des Transferplattenzufuhrmechanismus (21) die Transferplatte (92) zu einem Transferplatteneingangsbereich (101) auf einer Basis (10) bewegt; die Transferplatte (92) mehrere darauf gebildete Lösungsaussparungen (921) aufweist; ein Positionierungsschieber (31) eines Transferplattenpositioniermechanismus (30) sich zu dem Transferplatteneingangsbereich (101) bewegt und mit der in dem Transferplatteneingangsbereich (101) befindlichen Transferplatte (92) verbunden wird; der Plattenzuführer (213) des Transferplattenzufuhrmechanismus (21) sich zu der Position oberhalb des Positionierungsschiebers (31) bewegt, um die Transferplatte (92) an den Positionierungsschieber (31) zu übergeben; der Positionierungsschieber (31) die Transferplatte (92) zu einem Aufnahmebereich (102) auf der Basis (10) bewegt; ein Injektionsmechanismus (40) mehrere Lösungen (91) in die Lösungsaussparungen (921) der Transferplatte (92) injiziert; und
(b) Abgeben von Zellen oder Lösungen (91), wobei der Positionierungsschieber (31) die Transferplatte (92) zu einem Abgabebereich (103) auf der Basis (10) bewegt; eine Zellkulturplatte (94) mehrere Wells (941) aufweist; ein Abgabemechanismus (80) eine Pipette (93) zwischen einer Position oberhalb des Abgabebereichs (103) auf der Basis (10) und einer Position oberhalb der Zellkulturplatte (94) auf dem Positionierungsschieber (31) hin und her bewegt, um Flüssigkeit in jede der Lösungsaussparungen (921) in die Wells (941) der Zellkulturplatte (94) abzugeben.

9. - Zell- und Medikamentenabgabeverfahren nach Anspruch 8, wobei in dem Schritt (a), nachdem der Plattenzuführer (213) die Transferplatte (92) zu dem Transferplatteneingangsbereich (101) auf der Basis (10) bewegt hat, der Transferplattenzufuhrmechanismus (21) die unterste Ersatztransferplatte (96) zu dem Plattenzuführer (213) bewegt.

10. - Zell- und Medikamentenabgabeverfahren nach Anspruch 8 oder 9, wobei
in dem Schritt (a), nachdem der Injektionsmechanismus (40) Lösungen (91) in die Lösungsaussparungen (921) der Transferplatte (92) injiziert hat, eine gleitende Plattenabdeckung (34) Öffnungen der Lösungsaussparungen (921) der Transferplatte (92) abdeckt; und
in dem Schritt (b) sich die gleitende Plattenabdeckung (34) weg von der Transferplatte (92) bewegt, um eine der Lösungsaussparungen (921) freizulegen, bevor die Pipette (93) Flüssigkeit in die eine der Lösungsaussparungen (921) abgibt; nachdem die Pipette (93) Flüssigkeit in eine der Lösungsaussparungen (921) abgegeben hat, sich die gleitende Plattenabdeckung (34) zurück in Richtung der Transferplatte bewegt, um die Öffnungen der Lösungsaussparungen (921) vollständig abzudecken.

## Revendications

1. - Dispositif de distribution de cellules et de médicaments pour criblage de médicaments, **caractérisé par le fait que** le dispositif de distribution de cellules et de médicaments est configuré pour injecter de multiples solutions (91) dans de multiples évidements de solution (921) d'une plaque de transfert (92) ; ensuite, du liquide à l'intérieur de chacun des évidements de solution (921) est distribué dans de multiples puits (941) d'une plaque de culture cellulaire (94) avec une pipette (93) ; au moins une pointe de pipette (95) est reliée de manière amovible à une partie inférieure de la pipette (93) ; le dispositif de distribution de cellules et de médicaments comprenant :
une base (10) ayant sur celle-ci une zone d'entrée de plaque de transfert (101), une zone de réception (102) et une zone de distribution (103) ;
un mécanisme de service de plaque de transfert (21) monté sur la base (10) et adjacent à la zone d'entrée de plaque de transfert (101) ; le mécanisme de service de plaque de transfert (21) étant configuré pour accueillir la plaque de transfert (92) et apte à déplacer la plaque de transfert (92) jusqu'à la zone d'entrée de plaque de transfert (101) de la base (10), le mécanisme de service de plaque de transfert (21) ayant
un support d'empilement de plaques (211) formant un espace d'empilement ; l'espace d'empilement étant configuré pour accueillir de multiples plaques de transfert de rechange (96) qui sont empilées verticalement ;
une ouverture de chute de plaque (2111) formée dans un fond du support d'empilement de plaques (211) ; les plaques de transfert de rechange (96) étant aptes à tomber à travers l'ouverture de chute de plaque (2111) ;
deux pinces de blocage de plaque (212) ; chacune des pinces de blocage de plaque (212) étant montée sur un côté respectif de deux côtés opposés de l'ouverture de chute de plaque (2111) ; les deux pinces de blocage de plaque (212) étant aptes à se déplacer l'une vers l'autre pour empêcher les plaques de transfert de rechange (96) de passer à travers l'ouverture de chute de plaque (2111) ;
un dispositif d'alimentation en plaque (213) monté sous l'ouverture de chute de plaque (2111) et configuré pour recevoir les plaques de transfert (92) tombées à travers l'ouverture de chute de plaque (2111) ; le dispositif d'alimentation en plaque (213) étant mobile jusqu'à la zone d'entrée de plaque de transfert (101) de la base (10) ; le dispositif d'alimentation en plaque (213) ayant :
une ouverture de levage de plaque (2131) formée à travers deux côtés opposés du dispositif d'alimentation en plaque (213) ; et
un actionneur de levage de plaque (214) monté sous l'ouverture de levage de plaque (2131) et apte à se déplacer vers le haut pour faire saillie à travers l'ouverture de levage de plaque (2131) ;
un mécanisme de positionnement de plaque de transfert (30) monté sur la base (10) et adjacent au mécanisme de service de plaque de transfert (21) ; le mécanisme de positionnement de plaque de transfert (30) ayant :
un coulisseau de positionnement (31) configuré pour être relié à la plaque de transfert (92) ; le coulisseau de positionnement (31) étant monté de manière mobile sur la base (10) et correspondant de manière sélective en position à la zone d'entrée de plaque de transfert (101), à la zone de réception (102) ou à la zone de distribution (103), le dispositif d'alimentation en plaque (213) étant configuré pour se déplacer jusqu'à une position au-dessus du coulisseau de positionnement (31) pour transférer la plaque de transfert (92) au coulisseau de positionnement (31) ;
un mécanisme d'injection (40) monté sur la base (10) et adjacent au mécanisme de positionnement de plaque de transfert (30) ; le mécanisme d'injection (40) ayant :
de multiples têtes d'injection (41) ; chacune des têtes d'injection (41) étant en communication fluidique avec l'une respective des solutions (91) et étant mobile jusqu'à la zone de réception (102) de la base (10) ; le coulisseau de positionnement (31) étant configuré pour aligner l'un quelconque des évidements de solution (921) de la plaque de transfert (92) avec la tête d'injection (41) située dans la zone de réception (102) de telle sorte que ladite tête d'injection (41) injecte l'une des solutions (91) dans l'un correspondant des évidements de solution (921) ;
un mécanisme de positionnement de plaque de culture cellulaire (60) monté sur la base (10) et configuré pour être relié à la plaque de culture cellulaire (94) ; le mécanisme de positionnement de plaque de culture cellulaire (60) ayant :
un siège de positionnement (61) monté sur la base (10) et configuré pour accueillir la plaque de culture cellulaire (94) ; et
un module de positionnement primaire (62) monté sur le siège de positionnement (61) et configuré pour serrer la plaque de culture cellulaire (94) ; et
un mécanisme de distribution (80) monté sur la base (10) et ayant :
un siège de distribution (81) configuré pour fixer la pipette (93) et configuré pour commander des opérations d'aspiration et de décharge de la pipette (93) ; le siège de distribution (81) étant mobile par rapport à la base (10) et mobile en va-et-vient entre une position au-dessus de la zone de distribution (103) de la base (10) et une position au-dessus du mécanisme de positionnement de plaque de culture cellulaire (60) ;
le siège de distribution (81) étant configuré pour se déplacer jusqu'à une position au-dessus de la zone de distribution (103) pour introduire l'au moins une pointe de pipette (95) de la pipette (93) dans l'un quelconque des évidements de solution (921) de la plaque de transfert (92) et exécuter l'opération d'aspiration de la pipette (93) ; et
le siège de distribution (81) étant configuré pour se déplacer jusqu'à une position au-dessus du mécanisme de positionnement de plaque de culture cellulaire (60) et exécuter l'opération de décharge de la pipette (93) pour libérer la solution (91) aspirée dans l'au moins une pointe de pipette (95), dans l'un des puits (941) de la plaque de culture cellulaire (94).

2. - Dispositif de distribution de cellules et de médicaments selon la revendication 1, comprenant en outre :
un dispositif d'alimentation en pointe de pipette (70) monté sur la base (10) et ayant
une courroie transporteuse d'entrée (71) ; et
un module de positionnement de pointe (73) monté sur une extrémité de la courroie transporteuse d'entrée (71) ;
le siège de distribution (81) du mécanisme de distribution (80) étant mobile jusqu'à une position au-dessus du module de positionnement de pointe (73) du dispositif d'alimentation en pointe de pipette (70) et mobile vers le module de positionnement de pointe (73).

3. - Dispositif de distribution de cellules et de médicaments selon la revendication 2, dans lequel
le siège de distribution (81) du mécanisme de distribution (80) est pivotant autour d'un axe de pivotement (87) ; l'axe de pivotement (87) n'est pas parallèle à la verticale ;
le mécanisme de distribution (80) a en outre
un actionneur d'inclinaison (86) relié au siège de distribution (81) et commandant un angle de rotation du siège de distribution (81) autour de l'axe de pivotement (87).

4. - Dispositif de distribution de cellules et de médicaments selon la revendication 2, dans lequel
le dispositif d'alimentation en pointe de pipette (70) a en outre :
une courroie transporteuse de sortie (72) montée sous la courroie transporteuse d'entrée (71) ; la courroie transporteuse de sortie (72) et la courroie transporteuse d'entrée (71) fonctionnant dans des directions opposées ;
le module de positionnement de pointe (73) est apte à se déplacer vers le bas pour s'aligner avec une extrémité de la courroie transporteuse de sortie (72).

5. - Dispositif de distribution de cellules et de médicaments selon l'une quelconque des revendications 1 à 4, dans lequel
la plaque de culture cellulaire (94) a un couvercle supérieur (942) recouvrant de manière amovible les puits (941) ; et
le mécanisme de positionnement de plaque de culture cellulaire (60) a en outre
un dispositif d'ouverture de couvercle (63) monté au-dessus du siège de positionnement (61) ; le dispositif d'ouverture de couvercle (63) étant apte à se déplacer vers le haut et vers le bas, et apte à se déplacer latéralement ; le dispositif d'ouverture de couvercle (63) étant mobile jusqu'à une position au-dessus de la plaque de culture cellulaire (94) ; et
au moins une ventouse (64) montée sur une surface inférieure du dispositif d'ouverture de couvercle (63) et configurée pour adhérer au couvercle supérieur (942) de la plaque de culture cellulaire (94).

6. - Dispositif de distribution de cellules et de médicaments selon l'une quelconque des revendications 1 à 4, dans lequel
le coulisseau de positionnement (31) du mécanisme de positionnement de plaque de transfert (30) est apte à se déplacer vers le haut et vers le bas ; et
lorsque le coulisseau de positionnement (31) correspond en position à la zone d'entrée de plaque de transfert (101), le dispositif d'alimentation en plaque (213) du mécanisme de service de plaque de transfert (21) est mobile jusqu'à une position au-dessus du coulisseau de positionnement (31), et le coulisseau de positionnement (31) est apte à se déplacer vers le haut pour faire saillie à travers l'ouverture de levage de plaque (2131).

7. - Dispositif de distribution de cellules et de médicaments selon l'une quelconque des revendications 1 à 4, dans lequel le mécanisme de positionnement de plaque de transfert (30) a
un capot de plaque coulissant (34) monté de manière mobile sur le coulisseau de positionnement (31) et mobile jusqu'à une position au-dessus du coulisseau de positionnement (31) pour recouvrir des ouvertures des évidements de solution (921) de la plaque de transfert (92).

8. - Procédé de distribution de cellules et de médicaments pour criblage de médicaments, **caractérisée par le fait que** le procédé de distribution de cellules et de médicaments comprend les étapes suivantes :
(a) transférer des cellules ou des solutions (91), un mécanisme de service de plaque de transfert (21) ayant un support d'empilement de plaques (211), une ouverture de chute de plaque (2111), deux pinces de verrouillage de plaque (212), un dispositif d'alimentation en plaque (213) et un actionneur de levage de plaque (214) ; le support d'empilement de plaques (211) formant un espace d'empilement ; l'espace d'empilement étant configuré pour accueillir de multiples plaques de transfert de rechange (96) qui sont empilées verticalement ; l'ouverture de chute de plaque (2111) étant formée dans un fond du support d'empilement de plaques (211) ; les plaques de transfert de rechange (96) étant aptes à tomber à travers l'ouverture de chute de plaque (2111) ; les deux pinces de verrouillage de plaque (212) étant montées chacune sur un côté respectif de deux côtés opposés de l'ouverture de chute de plaque (2111) ; les deux pinces de blocage de plaque (212) étant aptes à se déplacer l'une vers l'autre pour empêcher les plaques de transfert de rechange (96) de passer à travers l'ouverture de chute de plaque (2111) ; le dispositif d'alimentation en plaque (213) étant monté sous l'ouverture de chute de plaque (2111) et configuré pour recevoir une plaque de transfert (92) tombée à travers l'ouverture de chute de plaque (2111) ; le dispositif d'alimentation en plaque (213) étant mobile jusqu'à la zone d'entrée de plaque de transfert (101) de la base (10) et ayant une ouverture de levage de plaque (2131) formée à travers deux côtés opposés du dispositif d'alimentation en plaque (213) ; l'actionneur de levage de plaque (214) étant monté sous l'ouverture de levage de plaque (2131) et apte à se déplacer vers le haut pour faire saillie à travers l'ouverture de levage de plaque (2131) ; le dispositif d'alimentation en plaque (213) du mécanisme de service de plaques de transfert (21) déplaçant la plaque de transfert (92) jusqu'à une zone d'entrée de plaque de transfert (101) sur une base (10) ; la plaque de transfert (92) ayant de multiples évidements de solution (921) formés sur celle-ci ; un coulisseau de positionnement (31) d'un mécanisme de positionnement de plaque de transfert (30) se déplaçant jusqu'à la zone d'entrée de plaque de transfert (101) et étant relié à la plaque de transfert (92) située dans la zone d'entrée de plaque de transfert (101) ; le dispositif d'alimentation en plaque (213) du mécanisme de service de plaque de transfert (21) se déplaçant jusqu'à la position au-dessus du coulisseau de positionnement (31) pour transférer la plaque de transfert (92) au coulisseau de positionnement (31) ; le coulisseau de positionnement (31) déplaçant la plaque de transfert (92) jusqu'à une zone de réception (102) de la base (10) ; un mécanisme d'injection (40) injectant de multiples solutions (91) dans les évidements de solution (921) de la plaque de transfert (92) ; et
(b) distribuer des cellules ou des solutions (91), le coulisseau de positionnement (31) déplaçant la plaque de transfert (92) jusqu'à une zone de distribution (103) sur la base (10) ; une plaque de culture cellulaire (94) ayant de multiples puits (941) ; un mécanisme de distribution (80) déplaçant une pipette (93) en va-et-vient entre une position au-dessus de la zone de distribution (103) sur la base (10) et une position au-dessus de la plaque de culture cellulaire (94) sur le coulisseau de positionnement (31) pour distribuer du liquide dans chacun des évidements de solution (921), dans les puits (941) de la plaque de culture cellulaire (94).

9. - Procédé de distribution de cellules et de médicaments selon la revendication 8, dans lequel, à l'étape (a), après que le dispositif d'alimentation en plaque (213) a déplacé la plaque de transfert (92) jusqu'à la zone d'entrée de plaque de transfert (101) sur la base (10), le mécanisme de service de plaque de transfert (21) déplace la plaque de transfert de rechange (96) la plus basse jusqu'au dispositif d'alimentation en plaque (213).

10. - Procédé de distribution de cellules et de médicaments selon la revendication 8 ou 9, dans lequel
à l'étape a), après que le mécanisme d'injection (40) a injecté des solutions (91) dans les évidements de solution (921) de la plaque de transfert (92), un capot de plaque coulissant (34) recouvre des ouvertures des évidements de solution (921) de la plaque de transfert (92) ; et
à l'étape (b), le capot de plaque coulissant (34) est déplacé à l'opposé de la plaque de transfert (92) pour découvrir l'un des évidements de solution (921) avant que la pipette (93) ne distribue du liquide dans ledit évidement de solution (921) ; après que la pipette (93) a distribué du liquide dans ledit évidement de solution (921), le capot de plaque coulissant (34) revient vers la plaque de transfert pour recouvrir entièrement les ouvertures des évidements de solution (921).
